# EUROPEAN PATENT APPLICATION

(11) **EP 3 811 922 A1**
(43) Date of publication of application: **28.04.2021**
(21) Application number: 19205134.0
(22) Date of filing: 24.10.2019
(51) Int. Cl.: A61K 8/22, A61K 8/38, A61K 8/81, A61P 1/02, A61Q 11/00, A61Q 11/02

(54) **KIT, SYSTEM AND METHOD FOR PREPARING AND USE OF A PEROXIDE-CONTAINING COMPOSITION**

(71) Applicant: Koninklijke Philips N.V., 5656 AG Eindhoven (NL)
(72) Inventor: MAO, Jingliang, 5656 AE Eindhoven (NL); GODDARD, Gregory Russ, 5656 AE Eindhoven (NL)
(74) Representative: Philips Intellectual Property & Standards

(57) **Abstract**

Provided is a kit or system (100) for preparing a peroxide composition with increased viscosity with a pH in the range of 7 to 12 in an almost instantaneous method. For example, the kit or system may be used to provide an almost instantly obtainable (hydro)gelled peroxide composition. The system comprises a first aqueous composition (102) comprising a peroxide agent. The first aqueous composition has a first pH of less than 7. The system further comprises a second composition (104) comprising a basic agent. The second composition is separate from the first aqueous composition. A pH induced thickener is included in the kit or system separate from the second composition. The peroxide composition is obtainable by combining the parts of the kit or system. The pH induced thickener is capable of increasing the viscosity of the peroxide composition upon subjecting it to the pH in the range of 7 to 12. The kit or system has sufficient basic agent for the peroxide composition to cause the pH in the range of 7 to 12. A method for preparation and use of the kit or system is also provided.

## Description

### FIELD OF THE INVENTION

This invention relates to systems, kits and methods for preparing a thickened peroxide composition from at least two separate compositions. The invention further relates to the peroxide composition and the use of these systems, kits and thickened peroxide composition for tooth whitening, disinfection and sanitization purposes such as disinfection for prevention or treatment of oral infections. The invention also relates to a method for preparing the peroxide composition.

### BACKGROUND OF THE INVENTION

Hydrogen peroxide, based on its oxidizing properties is conventionally used for sanitization, disinfection and tooth whitening, where with tooth whitening the natural tooth shade is restored or whitened beyond the natural tooth shade.

In oral applications peroxide compositions including hydrogen peroxide and/or carbamide peroxide are oftentimes used. The peroxide may, for example, be included in toothpastes, mouth rinses, chewing gums, paint-on films, etc. For whitening, peroxide containing gels with increased viscosity are often used. The gels may be brought in contact with teeth using a tray, plastic strip or other means. The gel, possibly together with the tray, strip or other means, may be obtained either over-the-counter (home use or take home) or from a dental professional (chair side).

The gels are commonly supplied in kits which include a pre-gelled polymer composition. Whilst this gel-like consistency facilitates application of such compositions to surfaces of e.g. dental pieces for whitening, supplying such pre-formed gels to the end user has proven problematic, because the rheological properties generally are subject to deterioration over time while they should remain stable during the product's shelf life which oftentimes can amount to a significant period of time, e.g. several days, weeks or months, before the kits are used.

The rheology development over time of the gel-like constituent may thus necessitate inclusion of stabilizing ingredients and/or refrigerated transportation (e.g. at 2-8 °C) and storage of the conventional kit, therewith increasing complexity and cost of formulating, storing and shipping as well as use of the conventional kits. Moreover, the inclusion of such additional stabilizing ingredients, such as specific thickeners, chelants, desensitizers, etc. in the formulation results in the constituent having higher osmolality, which is believed to lead to higher whitening sensitivity (Abd-Elmeguid A, Yu DC.; JADC. 2009; 75(1): 55; Pashley DH et al.; Inside Dentistry (supp). 2008 Oct: 4(9) (special issue)).

Furthermore, the increased viscosity of the preformed gels may cause it to be difficult to prepare, package and store. The relatively high viscosity may make for less efficient blending with other kit constituents such as e.g. the peroxide compound. Precisely controlled production conditions may thus be required.

In addition, visible gas, e.g. air, bubbles may be trapped in the pre-formed gel composition, which may detriment the appearance, and hence the perceived quality, of the kit when the polymer-containing constituent is supplied in a transparent compartment, such as a transparent syringe barrel. The gas bubbles, combined with the tackiness of the pre-formed gel, may also make accurate dosing of the composition difficult.

Thus, while such pre-formed gels may fulfil their intended purpose based on their viscous properties, further improvements are desired. In particular, it would be desirable to improve at least one of, but preferably more of: the ease of production, shelf-life, handling, dosing accuracy and appearance of such gel-based (teeth whitening) products.

### SUMMARY OF THE INVENTION

The invention as defined by the claims is aimed at providing improvement at least with regard to one or more of the aforementioned problems/disadvantages.

According to an aspect there is provided a system as claimed in claim 1. The system is a multi-composition system or multi-component system or multi-part system which upon mixing of amounts of each of the compositions or components or parts results in a desired specified peroxide composition for use. The system may be a kit of parts.

The inventors have recognized that, considering the aforementioned aim, a careful choice of multiple ingredients and their distribution over at least two separate components of a system of components to be mixed together to prepare a desired peroxide composition may be used to fulfill the aim.

The system is for preparing a peroxide composition having a composition pH in the range of 7 to 12. The peroxide composition can be obtained by combining or mixing the constituents of the system as elucidated herein after. The peroxide composition is a peroxide containing composition, i.e. a composition that comprises peroxide or a source of peroxide. The peroxide preferably comprises the peroxide agent. The peroxide agent preferably comprises or consists of hydrogen peroxide and/or a source of hydrogen peroxide such as carbamide peroxide.

Due, at least in part, to its pH induced thickener and pH in the range of 7 to 12, the peroxide composition has an increased viscosity at least with respect to the compositions or components it is made of and may have the form of a gel and in particular a hydrogel.

The system includes a first composition having a peroxide agent and water and has an acidic pH (first pH below 7). The peroxide agent preferably comprises or consists of hydrogen peroxide and/or a source of hydrogen peroxide such as carbamide peroxide.

The system also includes a second composition comprising a basic agent, which serves as a pH modifier in that when mixed with the first composition it causes the pH of the resulting peroxide composition to be less acidic than that of the first composition. Furthermore, the system comprises a pH induced thickener separate from the second composition. The pH induced thickener is an agent that provides an increase of viscosity (thickening) of an aqueous composition upon subjecting it to an increased pH. It thus is able to cause a thickening of the peroxide composition which is at pH in the range of 7 to 12. In the system the pH induced thickener is kept separate from the second composition. It is kept in solvent free and/or dispersion medium free form or in a composition having a solvent and/or dispersion medium when such medium has an acidic pH. When kept in such forms it provides little or no thickening of any system compositions. The amount of the basic agent is chosen so that any acidic constituents ending up in the peroxide composition become compensated to such extent that the resulting peroxide composition pH is in the required range.

The first quantity and second quantity of the first and second compositions and the amount of pH induced thickener are at least sufficient for a mixture of all three constituents to provide a peroxide composition as desired. In an embodiment the first quantity and second quantity provide all of the first and second composition present in the system.

While the system provides good handling during use and manufacture because of the low viscosity or solid state components in it, upon use, the system provides an almost instantly thickened peroxide composition with good viscosity. Therewith the system reduces one or more of the difficulties described herein before related to the pre-fabricated viscous peroxide gels. The thickening may occur in relatively short time span after or during mixing of system constituents and in some of the embodiments this may be almost instantly or instantaneously, which benefits applications in general and oral applications in particular.

Furthermore, the system has good shelf-life, because the peroxide agent in the first aqueous composition is kept at acidic pH. Peroxides generally are more stable at such pH. For example, a preferred peroxide agent comprises or consist of one or more of hydrogen peroxide and or hydrogen peroxide sources such as carbamide peroxide. While hydrogen peroxide is best kept at pH below 4, carbamide peroxide is best kept at pH below 6, but can also be kept below 5 or below 4. A preferred first pH is therefore in the range of 2 to 6, 2 to 5 or 2 to 4.

While stabilized in the system at low pH during storage, upon use of the system, the peroxide agent in the peroxide composition provides a good disinfection, whitening or sanitization effect. This is because in the peroxide composition the peroxide agent is exposed to the higher pH in the range of 7 to 12 where peroxide agents in general, and the hydrogen peroxide and its complexes (such as carbamideperoxide) in particular, are activated and have higher bleaching, whitening, or disinfection efficacy.

The second composition comprises or consists of a basic agent. The basic agent may comprise or consist of one or more basic compounds each capable of generating hydroxide ions (OH⁻) in aqueous environment or aqueous solution. The one or more compounds may be Bronsted bases or Arrhenius bases. They may be strong bases such as e.g. alkali metal hydroxides or alkali earth metal hydroxides or oxides. They may also be weak acids, such as e.g. alkali metal carbonates or acetates, or alkali earth metal carbonates or acetates. Preferably they are water soluble.

The second composition preferably comprises a solvent and/or a dispersion medium in which the basic agent is dissolved and/or dispersed. Preferably the second composition is an aqueous composition. Preferably it is a solution of basic agent with a second pH higher than 7.

Alternatively, the second composition may be solvent and dispersion medium free and thus comprise the basic agent in solvent-free and/or dispersion medium-free form. Since many of the bases mentioned herein generally are solids at standard conditions, a basic agent having one or more of such compounds is also a solid. This dry form of second composition is useful when a dilution of system ingredients, such as e.g. the peroxide agent, due to combination of the compositions of the system upon use is to be reduced or prevented. Sometimes a relatively high peroxide agent content (e.g. higher than 20 wt % hydrogen peroxide) is needed in the peroxide composition. For a system having a second composition including a solvent and/or dispersion medium and thus exhibiting a significant dilution upon use, peroxide agent content in the first aqueous composition would have to be even higher than this. It is however often desired to keep peroxide agent contents as low as possible and at least below 20 wt % as for example a 20 or higher wt. % hydrogen peroxide concentration is considered hazardous.

In an embodiment the pH induced thickener comprises or consists of one or more polymers each comprising monomer residues wherein at least a fraction of the monomer residues comprises acidic groups. Depending on pKa, at least a part of such acidic groups will be in protonated form at the first pH and in deprotonated form at the composition pH in the range of 7 to 12. The deprotonation provides charged sites in the form of conjugated bases of the acidic groups which may engage in polar interactions believed to be responsible for the thickening as explained hereinafter. Such polymers having acidic groups may be good ASE and/or HASE polymers as defined hereinafter.

The acidic groups comprise or consist of one or more groups chosen from carboxylic acid groups, sulfonic acid groups, and phosphoric acid groups. Particularly preferred are carboxylic acid groups. The groups of a particular type (e.g. carboxylic acid) may be all identical or different in the one or more polymers. For example, the difference may arise as a consequence of their binding to the polymer residues via different substituents or groups. This may be used to adjust acidity constants of such groups as known in the art. Many polymers having such groups, and in particular the carboxylic acid groups, are compatible with oral use and/or are environmentally accepted. They may have pKa values suitable for obtaining compositions of the specified pH for the respective compositions such as the specified first pH. The acidic groups may all have the same pKa, but may also have different pKa. Such polymers when dispersed or dissolved in any system composition may provide such composition with a pH lower than 7 (or even lower than 5) on their own, i.e. without other acidic agents added. Thus, the pH of any such system composition may not need any addition of other acidic or basic agents to adjust the pH to a specifically desired level. Conversely, if pKa values cause pH of compositions that differs from desired values, additional acidic or basic agents may be added.

In an embodiment, the pH induced thickener comprises or consists of one or more alkali-soluble emulsion (ASE) polymers and/or one or more hydrophobically-modified alkali-soluble emulsion (HASE) polymers. Such polymers are generally capable of forming viscous hydrogels with water under alkaline conditions, while they are solids when in dry form or form relatively low viscosity solutions and/or dispersions (e.g. lower than 1000 mPa·s) even in the presence of water under acidic conditions such as the first pH. The ASE and HASE polymers having the acidic groups as defined herein before (e.g the carboxylic acid groups) are preferred in this sense. Thus ASE or HASE polymers may provide examples of the one or more polymers each having monomer residues with at least a fraction of their monomers comprising acidic groups.

ASE and HASE polymers are advantageous as they are capable of dispersing relatively high content of ionic and non-ionic ingredients upon thickening such as the peroxide agents and other ingredients disclosed herein. Furthermore, many of the ASE and HASE polymers such as the acrylic and cellulosic based ones described hereinafter are oxidation resistant and thus may be combined in the peroxide containing (i.e. the first) composition.

HASE polymers are preferred over ASE polymers due to their improved thickening properties in terms of higher viscosities being attainable at similar concentration and/or faster thickening as well as lower viscosities being attainable at acidic conditions of the first composition and/or third composition (if present) of the system. These properties may be used to advantage to increase the peroxide content in the system and peroxide composition and/or to provide higher viscosity peroxide compositions. Higher viscosity is sometimes desired for bodily or oral application of the system or peroxide composition as the environmental temperature is then almost 40°C and viscosities are known to decrease with increasing temperature. Also mechanical action in the mouth for example by tongue must be resisted by the peroxide composition in order to increase resident time in the mouth. HASE polymers may also provide a higher thickening rate due to the additional thickening mechanism writ to ASE polymers, which mechanism is advantages for direct (e.g. without strips or trays) application of the peroxide composition in the oral cavity.

In an embodiment, the pH induced thickener comprises or consist of one or more polymers each having at least a fraction of monomer residues with acidic groups wherein the one or more polymers are acrylic copolymers. The acidic groups then preferably comprise or consist of carboxylic acid groups. An acrylic copolymer is a polymer having monomer residues of one or more acrylic acids (e.g. acrylic acid and/or methacrylic acid or other) and monomer residues of one or more acrylic acid esters or acrylates (e.g. methylacrylate and/or methylmethacrylate and/or or other). Acrylic co-polymers having ester groups with less than 6 carbon atoms provide suitable ASE polymers. Examples of acrylic ASE polymers are the commercially available Acusol™ 810 and Carbomer™ 940F. Acrylic co-polymers where at least part of the monomer residues of acrylic acid esters have a hydrophobic ester group with more than 6 carbon atoms, such as between 6 and 40 carbon atoms provide suitable HASE polymers. One example of an acrylic HASE polymer is the commercially available Acusol™ 820.

In an embodiment, wherein the pH induced thickener comprises one or more cellulose polymers having residues at least a fraction of which comprise hydroxyl groups. The hydroxyl groups may be spaced from the backbone by alkyl moieties attached to the cellulosic backbone such as with hydroxymethyl cellulose (HMC), hydroxyl ethyl cellulose (HEC) or hydroxypropyl cellulose (HMC). In this embodiment, the pH dependency of the thickening does not depend on deprotonizable groups (such as acidic groups) to produce i.e. charged groups. These polymers are preferably provided separate from the peroxide agent, i.e. separate from the first composition as described herein above.

In an embodiment, the pH induced thickener is comprised at least partly, but preferably entirely in the first composition. Preferably all of it is in the first quantity. Since the first pH is below 7, the pH induced thickener will not cause substantial viscosity increase. Thus, the system may be simplified, e.g. relative to the scenario where the first composition and the pH induced thickener are provided separately from each other in for example a third composition. This embodiment may provide a two-component or two-part system of only two compositions being the first composition and the second composition. It may be used with a simple two-barrel or dual-barrel syringe or two-compartment dispenser system as defined hereinafter. This system with pre-formed combination of the pH induced thickener and the peroxide agent in the one (the first) composition may result in these constituents being more intimately mixed or evenly distributed in this first composition as well as in the final peroxide composition. The minimized number of system compositions to be mixed may provide easier mixing of compositions upon use of the system.

In an embodiment, the system includes a third quantity of a third composition separate from at least the second composition and the pH induced thickener is either distributed over the first quantity and the third quantity or is included only in the third quantity to keep it separate from the first composition. The third composition can include a solvent and/or dispersion medium in which the pH induced thickener is dissolved and/or dispersed. The third composition preferably is an aqueous composition having a third pH lower than 7. The third pH may be the same as the first pH, but this is not needed.

Alternatively or additionally, the third composition and thus also the pH induced thickener may be solvent-free and/or dispersion medium-free. Adding the pH induced thickener in pure (e.g. dry solid) form is again advantageous in reducing the aforementioned dilution effect. Furthermore, unwanted thickening through swelling with solvent or dispersion medium will be reduced or prevented.

When the pH induced thickener is present in solid state, which may be realized for many polymers disclosed hereinafter, it may even be mixed together with a basic agent provided the basic agent is also in solid state. No thickening can occur due to lack of solvent and/or dispersion medium. In yet a further variant, when the pH induced thickener is based on a compound or polymer having acidic groups (such as the ones disclosed herein after), it may be provided in salt form in which it has reacted with a base such that the conjugated base of the salt may provide at least part of the basic agent of the system. In both cases the thickener is thus part of the second composition that is solvent and/or dispersion medium free. Hence, again a simple kit of only two compositions can be made.

Having the pH induced thickener separate from the first composition, e.g. in the third composition or as part of the second composition that is solvent and/or dispersion medium free, is advantageous when the pH induced thickener is chemically unstable against the peroxide agent. In such case an improved shelf-life may be realized when compared to a system in which pH induced thickener is combined with the peroxide agent.

In a preferred embodiment, all compositions of the system comprise a solvent and/or dispersion medium and they are preferably all aqueous compositions thus comprising water. Combination of system compositions in liquid form is in general easier and/or faster to accomplish with more uniform and faster thickening occurring upon use of the system as compared to a situation where one or more of the ingredients has to be combined in solid state form. Furthermore, improved homogenous distribution and/or mixing of ingredients of each of the system compositions may be achieved. In such embodiment the pH induced thickener is preferably present in the first composition so that the system may only have two compositions for mixing.

In any of the systems defined the first quantity and the second quantity are sufficient to mix them in a ratio of the first quantity to the second quantity in a range of 10 to 0.1, preferably in the range of 5 to 0.2, or most preferably in the range of 5 to 0.8. Such mixing ratios provide easy to use systems. The quantities may be measured in volume or weight but preferably are measured in weight. Such mixing ratios provide a good balance of necessary quantities to be mixed and convenient use with a multi barrel syringe or the dispenser disclosed herein after.

Preferred variants of the system defined herein are those wherein the first composition comprises a solvent and/or dispersion medium and has a viscosity lower than 1000 mPa·s, preferably lower than 500 mPa·s, most preferably lower than 100 mPa·s at standard conditions. Such low viscosities improve the manufacture and use of the systems considerably. For example, a viscosity of less than 100 mPa·s is especially desired since such component has a flowability approaching that of water. The pH induced thickeners in the form of HASE polymers disclosed hereinafter may be particularly suitable to provide such low viscosities in the system e.g. when part of the first composition.

In an embodiment, the amount of pH induced thickener is chosen to cause the peroxide composition to have a viscosity higher than 50000 mPa·s, more preferably higher than 100000 mPa·s at standard conditions. Such high viscosities provide stable peroxide compositions which is especially useful in oral applications where mechanical forces caused by e.g. tongue may reduce retention time of the peroxide composition in the mouth. The pH induced thickeners comprising ASE and in particular HASE polymers are suitable for achieving such values instantly already at modest concentrations in the peroxide compositions and often almost instantly as will be elucidated herein after.

In embodiments the first pH is in the range of 2 to 5 and/or, when the system comprises the third composition having a third pH, the third pH is in the range of 2 to 5. The carboxylic acid type polymers such as for example the ASE and HASE polymers are suitable in this respect.

The system is preferably designed such that the composition pH is in the range of 7 to 12 for reasons explained herein before. For sanitization a composition pH in the range of 8 to 10 may be used. When the system is for use on the body of human or animal subjects such as for oral use as with oral disinfection or whitening of dental pieces, the composition pH is preferably in the range of 7 to 9.5 or more preferably between 7 to 9, or more preferably between 8 and 9 to be better body compatible and still provide good effectiveness and/or have sufficient viscosity. The first and second quantities and amounts of pH induced thickener and base are then chosen to result in such values. For example the, to be mixed, first and second quantity may be fixed as the contents of a dual barrel syringe as described herein after.

The system is preferably for preparing a peroxide composition for oral use such as for oral disinfection and/or tooth whitening. In such case the system may comprise one or more additives selected from: a tooth desensitizing agent, a tooth remineralizing agent and a flavoring agent. The one or more additives are for combining with the other respective compositions and the pH induced thickener of the kit to obtain the peroxide composition. The presence of any one of such additives may enable tuning of the peroxide composition and system for oral use with respect to taste, pain sensation and/or tooth repair. The desensitizing agent and the tooth remineralizing agent may respectively assist in enhancing comfort and protection of teeth during the oral use. The flavoring agent may trigger the sense of taste and/or smell, thereby to provide a better taste sensation during prolonged oral use. The flavoring agent may, for instance, add taste to an otherwise flavorless composition, or may be used to mask any tastes or odors which are less desirable from the point of view of the user of the system or peroxide composition.

Any one of these one or more additives may be present in the system in one or more further separate compositions. Preferably they are for example combined in the third composition (when present in the system) to not expose them to conditions such as basic (in the second composition) or acidic and/or oxidizing of the first composition. Preferably they are combined in one further system composition and this limits the number of compositions of a system to 3. Alternatively, the one or more additives are comprised with the first and/or second composition. This limits the number of components of a system to 2 and thus provides for a simpler system. If sensitive to wards oxidation and or acidic conditions, the flavoring agent is preferably not present in the first composition. If sensitive towards basic hydrolysis, the flavoring agent is not preferably not present in the second composition. In such cases shelf life of the system may be improved.

In embodiments the desensitizing agent comprises potassium ions and/or a source of potassium ions such as a potassium salt. Potassium ions are believed to desensitize nerve fibers blocking the neural transmission. For example potassium ions and/or a potassium ion source may be present in the first composition and/or the second composition. When present in the second composition, they may be present in the form of e.g. potassium hydroxide, carbonate or potassium nitrate.

In an embodiment the desensitizing agent comprises or consists of one or more compounds for plugging dental tubules. For example, such compounds may be any one of: Sodium fluoride, Stannous fluoride, Strontium chloride, Silver diamine fluoride, Potassium oxalate, Calcium or strontium phosphate, Calcium or strontium carbonate. Most of these such as the phosphates and carbonates may also serve as remineralization agents.

In an embodiment, the system comprises calcium ions or a source of calcium ions such as a calcium salt and/or strontium ions or a source of strontium ions such as a strontium salt and monobasic, and/or dibasic and/or tribasic phosphoric acid ions (phosphoric ions) or a source of such ions. Preferably the calcium ions, strontium ions or their sources are comprised within the first composition and then preferably in a form (e.g. in chloride or nitrate salts) soluble in an aqueous composition. The phosphoric ions or their source are preferably comprised within the second component. Separation of the phosphoric ions and their sources from the calcium or strontium ions or their sources may prevent the crystallization and precipitation of calcium or strontium phosphates during shelf life of the system, while upon mixing of the compositions upon use of the system, they will become combined to form calcium phosphate (CP) and/or strontium phosphate (SP) in crystalline or preferably amorphous (ACP and ASP) form in the peroxide composition. The calcium and strontium phosphates may serve as agents of remineralization or desensitization.

A suitable flavoring agent may, for instance, include natural peppermint oil, or artificial sweeteners preferably non-nutritional such as saccharine or sucralose which may provide the user with a sense of cleanliness and freshness when using the kit, particularly when the composition is applied to the dental pieces to be whitened.

In an embodiment, the system comprises one or more compartments for storing any of the systems compositions ingredients. The compartments may be part of individual containers, bottles or syringes. The system may have a vessel or other chamber, in which to combine the ingredients of the system for forming the peroxide composition. The device may have a means for mixing such as spatula.

In an embodiment, the system comprises a multi-compartment syringe, said syringe including: a first compartment containing the first quantity of the first composition, a second compartment containing the second quantity of the second composition, and an optional third compartment for comprising the third quantity of the third composition when part of the system; and a mixing compartment attachable to or attached to the multi-compartment syringe for receiving and mixing the respective quantities and the amount of pH induced thickener and having an outlet for permitting the resulting peroxide composition to exit the syringe upon the use of the syringe.

The syringe comprises plungers for forcing the components out of the respective compartments and into and through the mixing compartment. The plungers may be designed to be operated synchronously for example since they are mechanically connected to each other. The syringe may provide a convenient means of storing, shipping and use of the system. It may also provide a simple way of ensuring a desired ratio of quantities of the compositions to be mixed to arrive at the desired peroxide composition. Such quantities can be those as disclosed herein.

The mixing compartment may have a channel arrangement for mixing the contents of the respective compartments of the syringe. The channel may have fixed mixing blades for this purpose. Such channel arrangement can be for mixing of fluidic compositions such as solutions, emulsions or dispersions, but may also be arranged for mixing of such fluidic compositions with one or more solid compositions. This will depend on the nature of the compositions to be mixed. The inlet may have two, three, four sub-inlets one for each of the compositions to be mixed. The mixing part may be located in the tip of the syringe and may be integrated in the syringe, or attachable or attached to the syringe.

In an embodiment of a particularly simple system, the syringe comprise only two compartments being the first and second compartments. Any of the ingredients of the system compositions is then present in one or more of the first and second quantities. This provides a simple two-barrel syringe system.

In the case the pH induced thickener is included in the system a third barrel of the syringe may be present for comprising the third composition.

The mixing part may be employed to first combine the first composition with the pH induced thickener (for example as part of the third composition) before the resulting mixture is combined with the second composition in the mixing part.

In an embodiment, the system comprises a dispensing system for dispensing the peroxide composition which includes a mixing unit, a user interface and a selection module to control the mixing unit. The user interface combined with the selection module provides a dispenser system which, when loaded with compositions of the system, allows user definition of a dispensed peroxide composition. For example the user may need a peroxide composition for a particular use (e.g. oral or regular disinfection), or with a particular peroxide content (for mild or strong disinfection); or with a particular alkaline composition pH (lower for oral application) and/or flavor and/or smell of the peroxide composition (for oral application); or with a particular viscosity. Such parameter may then be used to determine mixing ratios of the quantities of the compositions of the system.

The system preferably includes the individualized or separate containers, bottles or other vessels having the compositions of the system as described herein before. Preferably these take the form of replaceable cartridges for the dispensing system.

The dispensing system may have a memory accessible by the dispenser system where the memory is for storing data on contents of systems compositions from which data may be retrieved to calculate the required quantities to be mixed in order to arrive at the required user preferences for the particular parameters.

The user interface may enable the user to select parameters which dictate the nature of the composition ultimately dispensed by the system. For example, the consistency of the composition may be controlled by the pH induced thickener included in the composition and/or the pH of the composition. According to the user selections, the selection module may provide an accurate and efficient means of dispensing the respective compositions which constitute the peroxide composition. In particular, the system may, for instance, enable flavor selection, e.g. when the user has opted and input to the system that the composition is intended for tooth whitening. In a simple example, the user may select either to include the flavoring agent in the composition, e.g. by opting to include the second separate part in the composition, or not to include the flavoring agent in the composition. In a more elaborate example, the user may select from different flavoring agents. The user interface may, for instance, take the form of a touchscreen to enable user-friendly parameter selection.

In an embodiment, the system is for providing the peroxide composition for use as an orally applicable tooth whitening composition or a disinfectant composition in the prevention or treatment of oral infections.

Thus the system may be used such that the peroxide composition is an orally applicable tooth whitening composition or a disinfectant composition for use in the prevention or treatment of oral infections.

In case the system is used for bodily and in particular oral application, such as for oral disinfection or tooth whitening, all components and constituents of such system are preferably compatible with such use and this means that they are substantially non-toxic and/or or accepted for oral, pharmaceutical or cosmetic use.

In such oral application the system preferably is for preparing a peroxide composition having a composition pH in the range of 7 to 9.5.

In an aspect there is provided a peroxide composition having a composition pH in the range of 7 to 12, preferably in the range of 7 to 9.5, comprising a peroxide agent, water, a basic agent and an amount of a pH induced thickener capable of thickening an aqueous composition upon an increase of the pH of this aqueous composition from an acidic to an alkaline value.

The peroxide composition is preferably obtainable or obtained by combining or mixing the first quantity of the first composition, the second quantity of the second composition and the pH induced thickener as defined in any of the system described herein.

According to yet another aspect there is provided a method as claimed. Combining the compositions as defined in any one of the system by a user is a convenient way of providing a peroxide composition with advantages similar to those described for the systems herein before.

In an embodiment, the step of combining comprises preparing an intermediate composition by combining the pH induced thickener with the first quantity of the first composition and combining the intermediate composition with the second quantity of the second composition. The step of forming an intermediate composition may permit effective, e.g. intimate, mixing of the ingredients contained in the first composition, with the pH induced thickener. Subsequent mixing of the intermediate composition with the second composition may afford the peroxide composition in situ in which the ingredients of all composition are effectively mixed with each other. This may be advantageous for mixing in systems having the pH induced thickener separately as when present in a third composition separate from the first composition.

The method can have a further step of applying the peroxide composition to one or more surfaces of an object to be treated with the peroxide composition, such as for example of a human or animal subject, and/or of an oral cavity of a human or animal subject, and/or of a dental piece within an oral cavity of a human or animal subject.

In the system, peroxide composition or method, the use as oral disinfectant in the prevention or treatment of an oral infection may comprise treatment of one or more infections such as gingivitis, periodontitis, peri-implant mucositis and peri-implantitis.

The system and method are designed such that the composition pH of a peroxide composition is in the range of 7 to 12. The first and second quantities may provide the entire first component and entire second component present in a system.

For sanitization a composition pH in the range of 8 to 10 may be used as a less corrosive alternative. When the system is for use on the body of human or animal subjects such as for the oral applications referred to herein, the composition pH is preferably in the range of 7 to 9.5 or in the range of 7 to 9, or more preferably in the range of 8 to 9 to be better body compatible and still provide good effectiveness of oxidizing action and/or provide a peroxide composition with sufficient viscosity.

Any of the features of systems may be used to define the uses, methods and peroxide compositions as disclosed herein and vice versa, and this may provide the concomitant benefits to the peroxide compositions, uses and methods as disclosed herein.

### BRIEF DESCRIPTION OF THE DRAWINGS

Embodiments and examples of what is claimed are described in more detail and by way of examples with reference to the accompanying schematic drawings, wherein:
FIG. 1 depicts a kit or system according to an embodiment;
FIG. 2 depicts a kit or system according to another embodiment;
FIG. 3 depicts a kit or system according to yet another embodiment;
FIG. 4 depicts a block diagram of a system according to an embodiment;
FIG. 5 depicts a block diagram of a system according to another embodiment; and
FIG. 6 depicts a flowchart of a method according to an embodiment.

### DETAILED DESCRIPTION OF THE EMBODIMENTS

The detailed description and specific examples, while indicating exemplary embodiments of the kits, systems, methods and uses, are intended for purposes of illustration only and are not intended to limit the scope of what is claimed. These and other features, aspects, and advantages of the claimed kits, systems, methods and uses of will become better understood from the following description, appended claims, and accompanying drawings. The Figures are schematic and not drawn to scale. The same reference numerals are used throughout the Figures to indicate the same or similar parts.

Before describing examples in detail the meaning of a few terms will be explained.

The pH induced thickener is capable of thickening of a composition upon increasing the pH of that composition. In particular it is capable of thickening of an aqueous composition. It may have other functions too, but it at least provides the thickening property.

The term "thickening" a composition means increasing the viscosity of that composition.

The term "in the range of' as used to indicate a range of values of parameters such as pH, amounts or contents and viscosity values is meant to include the values of the range limits unless otherwise specified. Thus, e.g. a pH in the range of 7 to 12 is meant to specify that the pH may be 7, 12 or in between these values.

The term "composition" refers to an entity having one or more constituents e.g. as in a pure agent or compound or a mixture of agents or compounds.

The term "acidic agent" refers to one or more compounds that when dissolved or dispersed in neutral water having a pH of 7 causes the water to have a pH lower than 7. It may refer to Bronsted or Arrhenius acid.

The term "basic agent" refers to one or more compounds that when dissolved or dispersed in neutral water having a pH of 7 causes the water to have a pH higher than 7.

The term "agent" may refer to one or more compounds.

The term "solvent" may refer to one or more pure solvents.

The term "dispersion medium" may refer to one or more liquids.

The term "aqueous composition" may refer to a composition having a solvent and/or dispersion medium that comprises at least 20 % by weight (wt. %) of water. Preferably the solvent and/or dispersion medium has at least 30, or 40 wt. % of water. More preferably the water content is at least 50 or 60 or 70 wt. %. In some embodiments the water content is at least 80, 90, or even 95 wt. %. Ultimately the solvent and/or dispersion medium may be only water.

The term "solvent-free" or dispersion medium-free means that there is less than 5 wt. % solvent and/or dispersion medium. Preferably there is less than 1 wt. % of solvent and/or dispersion medium. Most preferably there is substantially no solvent and/or dispersion medium.

The term "hydrophobic group" means a group that is not attracted or that is even repelled by a mass of water. Such group, does not dissolve in water but may form clusters together, forming micelles. A hydrophobic group in general is an apolar group not capable of substantial hydrogen bonding.

The term "dental pieces" includes teeth, caps, crowns, dentures, etc.

When components are specified to be "separate" or "separated" from each other, they are physically separated.

Specification or definition of parameters are with regard to standard conditions unless otherwise specified. Standard conditions means ambient pressure of 1 atmosphere (1 atm) and room temperature (e.g. 20 °C).

Whenever a content is expressed in % by weight (wt. %), conversion of such values into other content units such as moles per weight or mole per volume or vice versa is done using the relevant molar weights and densities as is usual in the art.

Any suitable analytical techniques known in the art may be employed for analyzing chemical compositions such as those defined in this application. For example, determination of a pH of any composition may be done by dipping a suitably calibrated pH probe into the relevant composition, or by titration, or by use of litmus paper or the like. Furthermore, determining a content of peroxide agent may be done using for example redox titration methods suitable for such purpose. Determination of type of ions present and/or their contents in compositions may be done using for example spot test methods for tracing types of ions or flame ionization techniques. E.g. phosphorus, calcium strontium and potassium ions may be determined in this way. Organic molecules may be analyzed using chromatography, NMR, IR, mass spectroscopy or other spectroscopy methods as is usual. Viscosity measurements may be carried out in many ways such as for example with Brookfield viscometer. As long as the same method is used to generate data to be compared, these can all be used to verify whether compositions fulfill a claims definition. Viscosity values mentioned herein refer to Brookfield viscosities measured at about 0.6 rpm and standard conditions unless otherwise specified. All such methods are well-known per se and will not be further described herein for the sake of brevity only.

In summary the disclosed are kits or systems (100) for preparing a peroxide composition with increased viscosity with a pH in the range of 7 to 12 in an almost instantaneous method. For example, the kit or system may be used to provide an almost instantly obtainable (hydro)gelled peroxide composition. The kit comprises a first aqueous composition (102) comprising a peroxide agent. The first aqueous composition has a first pH of less than 7. The kit further comprises a second composition (104) comprising a basic agent. The second composition is separate from the first aqueous composition. A pH induced thickener is included in the kit or system separate from the second composition. The peroxide composition is obtainable by combining the parts of the kit or system. The pH induced thickener modifier is capable of increasing the viscosity of the peroxide composition upon subjecting it to an increasing pH such as a pH in the range of 7 to 12. The kit or system has sufficient basic agent for the peroxide composition to cause the pH in the range of 7 to 12. A peroxide composition, method for preparation of the peroxide composition and use of the kit or system are also provided.

In this disclosure the system may have the form of a kit of parts.

With the system, pre-forming of viscous compositions in a system can be minimized or avoided since the entirety of the pH induced thickener provided in the system is in a state preventing it to exert its thickening property. Therewith any problems associated with producing, storing, transporting and/or dosing may be addressed. At the same time, directly upon use, the mixing of the components leads to thickening of the resulting peroxide composition with all advantages associated for handling during treatment.

The system comprises at least two mutually separated compositions. In some embodiments there may be more than two compositions such as three, or even four mutually separated compositions. In particular preferred embodiments the system comprises only three or preferably even only two compositions. All compositions of the system together include all ingredients for the peroxide composition to be made with the system. The third composition may be used for various purposes such as separation of basic agent from pH induced thickener and/or separate provision of additives to the system as will become apparent herein below. Having less compositions provides a simpler system which may be advantageous in terms of cost, ease of manufacture and use. A system having only three and preferably only two compositions may be advantageously used with a three or two-barrel syringe as described herein after.

The pH induced thickener may be present in the system in several ways. In a first example the pH induced thickener is part of the first aqueous composition. The modifier may be dissolved and/or dispersed. A dispersion agent and/or surfactant agent may be present. The aqueous composition may be an emulsion. Since the first pH is below 7 substantial viscosity increase will not be caused by the thickener. In principle, even with further constituents added to the system as explained herein after, this embodiment may provide a system of only two compositions being the first aqueous composition and the second composition. This may be advantageously used with a two-barrel syringe as defined herein after. Furthermore, a more effective, e.g. intimate, mixing or even distribution of the pH induced thickener with other ingredients (e.g. the peroxide agent) in the first aqueous composition may be achieved. Furthermore, since a number of system constituents are thus already premixed before use of the system, the final combination with a further composition is improved.

In another example, the pH induced thickener is kept separate from the first composition for example as part of a third composition. Such third composition can include a solvent and/or dispersion medium in which the pH induced thickener is dissolved and/or dispersed. The third composition preferably is an aqueous composition having a third pH lower than 7. Preferably the third pH is chosen to be lower than any one of the following values: 6, 5, 4 or 3. The third pH may be higher than or equal to 1 or 2. The third pH may be in the range of 2 to 7, 2 to 6, or 2 to 4. The third pH may be the same as the first pH. The third composition is separate from the second composition.

Alternatively or additionally, the third composition and therewith the pH induced thickener may be provided in the system in solvent-free and/or dispersion medium-free condition. Many pH induced thickeners, such as for example certain polymers described herein after, are in solid state at standard conditions. This is again advantageous in relation to reducing the aforementioned dilution effect upon combining of system compositions, while unwanted thickening through swelling with solvent or dispersion medium is still reduced or prevented.

In a variation of the dry form of the pH induced thickener, it may be combined or mixed with a dry solid state basic agent. Even though a chemical reaction could theoretically take place, in solid state such reactions may be rather slow. In yet a further variant, when the pH induced thickener is based on a compound or polymer having acidic groups (such as the ones disclosed hereinafter), it may be provided in salt form through reaction of the acidic groups with the basic agent. The conjugated base of the acidic groups present in the salt may provide at least part of the basic agent.

In the embodiment with the solvent and/or dispersion medium free third composition, it may be preferred to have a solvent and/or dispersion medium present in the system to pre-form a third (e.g. aqueous) composition by adding to it the solvent and/or dispersion medium, before this third composition is combined with the first aqueous composition or second composition. It may be first mixed with the first composition before the result is mixed with the third composition. The pre-formed third composition may have a constitution as described herein before for the third composition. In this way a more effective, e.g. intimate, mixing or even distribution of the pH induced thickener with other ingredients (see hereinafter) such as e.g the peroxide agent can be achieved when the pH induced thickener is kept in dry form. To this end dispersants may also be added to the respective compositions.

In a preferred configuration the first and/or second quantities are both aqueous compositions. The second composition may be a solution or an emulsion or dispersion. A dispersion agent and/or surfactant agent may be present if needed. Since the second pH is below 7 substantial viscosity increase will not be caused by the pH induced thickener when in contact with solvent or dispersion medium. Having the pH induced thickener in dissolved and/or dispersed form instead of in dry form may provide improved mixing properties, e.g. more homogeneous and/or more intimate and/or faster and/or may provide easier packaging and handling in the system. The improved mixing may lead to faster thickening and/or more homogeneous viscosity properties of the peroxide composition. An increased water content may aid and facilitate swelling of the pH induced thickener as for many if not all of the pH induced thickeners the thickening mechanism is based on water swelling. Good examples of these are the polyacrylic acid based ASE and HASE polymers and the cellulosic polymers described herein after.

Solvents or dispersion media may be pharmaceutically or cosmetically accepted when the system is to be used for bodily application or oral application. The solvent or dispersion medium of any one of the compositions or quantities of compositions can comprise or consist of water. For example there may be more than any one of the following amounts of water (measured in wt. % of the total weight of the respective composition): 5, 10, 15, 20, 25, 30, 40, 45, 50, 55, 60, 65, 70, 75, 80, 85, 90. The solvent and/or dispersion medium may include other solvents than water such as one or more alcohols such as ethanol, glycerol, propylene glycol or other. Alcohols, generally mix well with water and may improve the solubility of pH induced thickener or pH adjuster or other system ingredients. Also they may improve the disinfectant working of the peroxide composition in a synergistic way as they are known to break open cells.

The system may be used for various purposes, e.g. bodily and/or oral disinfection, tooth whitening, or sanitization or other. Each of these applications may entail use of specific ranges of quantities of compositions, amounts of ingredients therein, their pH as well as types of ingredients as will become apparent herein after.

It will be readily appreciated by the skilled person that the relative quantities of compositions in a system will be, at least to some degree, dependent on the nature and amounts of the various ingredients present in the respective compositions, the operative consideration being that the final peroxide composition should have the composition pH specified in a desired range of 7 to 12 with a peroxide content in a desired specified range and a particular final viscosity in a desired range.

A first guide to design of a system of desire includes the following considerations or steps that do not necessarily need to be followed in the described order.

From a desired quantity (volume or weight) of peroxide composition the amount of pH induced thickener needed to provide such composition with a desired viscosity is determined. Also, the amounts of peroxide and/or its sources and possible additives etc. are determined. Taking into account any ratios with which quantities of compositions of a system are mixed to arrive at a particular peroxide composition, the amounts of ingredients in the different compositions of the system can be determined. When appropriate, dilution, occurring upon mixing of system compositions, is taken into account according to the usual methods known to those skilled in the art. Calculation may be checked by testing the composition and for example a final composition pH may be measured and possibly corrected when not at spec. The correction may be done e.g. by changing (adding or removing) the amount of base if needed.

The peroxide agent may comprise or consist of one or more peroxide compounds. Preferably the peroxide agent comprises hydrogen peroxide (H₂O₂) and/or a source of hydrogen peroxide. Suitable sources of hydrogen peroxide are its complexes for example with urea (as in carbamide peroxide with the molecular formula H₂O₂-CO(NH₂)₂). Hydrogen peroxide and carbamide peroxide, both individually and in combination, have been shown to be effective disinfection and tooth whitening agents. They are orally acceptable. Other peroxide compounds can be used additionally or alternatively. Examples include but are not limited to: peroxides in solid form including alkali or alkaliearth metalperoxides, or peracids, such as e.g. peracetic acid. The peroxide containing component may include, for example, metal ion free peroxide compounds. Examples of metal ion free peroxide compounds include hydrogen peroxide and organic peroxides including urea peroxide (carbamide peroxide), glyceryl peroxide, benzoyl peroxide and the like.

The content of peroxide agent in any composition is expressed in molality, i.e. in moles of peroxide group/ion per gram (M_{pg}/g) of composition. This allows to account for the different molar weights and number of peroxide group/ions per peroxide compound for calculation of content based on weight percentages. Thus, e.g. in a composition there may be 0.00294 M_{pg}/g peroxide agent. This translates into 10 wt. % of hydrogen peroxide as hydrogen peroxide has Mw= 34.01 g/mol and one peroxide group per molecule, while it translates into 28 wt. % of carbamide peroxide as carbamide peroxide has Mw=94.07 g/mol and one peroxide group per molecule.

The peroxide content in the peroxide composition preferably is lower than ∼0.0147 M_{pg}/g, (∼50 wt. % H₂O₂). Higher contents of peroxides are generally considered hazardous. The peroxide content in the peroxide composition is preferably lower than any one of the following values (corresponding weight percentage values for H₂O₂ are between parentheses) for home use purposes: 0.0132 M_{pg}/g (45 wt. % H₂O₂), 0.0118 M_{pg}/g (40 wt. % H₂O₂), 0.00882 M_{pg}/g (30 wt. % H₂O₂), 0.00588 M_{pg}/g (20 wt. % H₂O₂), 0.00294 M_{pg}/g and (10 wt. % H₂O₂). The content can be in the range of 0.00588 M_{pg}/g (20 wt. % H₂O₂) and 0.000882 M_{pg}/g (3.0 wt. % H₂O₂) or in the range of 0.00294 M_{pg}/g (10 wt. % H₂O₂) and 0.00147 M_{pg}/g (5.0 wt. % H₂O₂). This may be e.g. suitable for home use tooth whitening purposes. Contents higher than 0.00294 M_{pg}/g (10 wt. % H₂O₂) are useful for chairside (at the dental professional) whitening purposes. These contents can be in the range of 0.00294 M_{pg}/g (10 wt. % H₂O₂) and 0.0118 M_{pg}/g (40 wt. % H₂O₂) or in the range of 0.00294 M_{pg}/g (10 wt. % H₂O₂) and 0.00588 M_{pg}/g (20 wt. % H₂O₂), or in the range of 0.00588 M_{pg}/g (20 wt. % H₂O₂) to 0.0118 M_{pg}/g (40 wt. % H₂O₂). Such higher contents give shorter treatment times and/or better whitening effect, but are also more dangerous in use. Peroxide contents lower than any of the following values 0.00147 M_{pg}/g (5 wt. % H₂O₂), 0.000588 M_{pg}/g (2 wt. % H₂O₂), or 0.000294 M_{pg}/g (1 wt. % H₂O₂) may be appropriate when the system is to be applied, for instance, in a fresh instant whitening toothpaste application. Corresponding range values in wt. % carbamide peroxide or any other peroxide or a mixture of compounds can be calculated as indicated herein above from the peroxide agent molalities. In the above ranges, the contents refer to concentrations regardless of whether the peroxide agent is in dissolved and/or dispersed state. It will be clear that for a composition where a dissolved peroxide agent is in a dissolved state, a range is maximized by the maximum solubility of that agent in the relevant solvent.

The pH induced thickener comprises or consists of one or more compounds that have chemical groups responsive to the change of pH from less than 7 to a pH in the range of 7 to 12 by invoking an increase of viscosity of the medium they are part of.

The one or more compounds preferably are oligomers or polymers. The polymers may be homo-polymers having a single type of monomer residue or co-polymers having multiple different monomer residues. The polymers may be regular or random co-polymers. The polymers may be ter-polymers. The polymers may be crosslinked or not.

The chemical groups are preferably distributed along at least part of a, but preferably along an entire backbone of a polymer. The chemical groups may be part of the backbone, directly attached to the backbone with a direct chemical bond, or may be attached via spacers of e.g. saturated hydro carbon chain moieties. Preferably such spacer has less than 5 carbon atoms or even less than 3 carbon atoms. Thus a polymer may have monomer residues part of which or all of which comprise the chemical groups.

A particularly suitable class of polymers comprises as the chemical groups acidic groups which depending on their pKa values may be at least partly deprotonated at the pH in the range of 7 to 12. The acidic groups have pKa values such that a larger fraction is deprotonated at pH higher than 7 than at pH below 7. An acidic group is considered fully protonated in a composition of pH equal to or lower than pKa -2. Conversely the group is considered fully deprotonated in a composition of pH equal to or higher than pKa + 2. Preferably the pKa values of the acidic groups are lower than any one of the following values: 7, 6.5, 6, 5.5 or even 5. A pKa that is at most equal to the second pH of the second component may ensure a surplus of acidic groups being in the protonated form, since around 50% of acidic groups will be deprotonated at pH=pKa. The acidic groups can be chosen to have pKa values in the range of: 2 to 7, or 3 to 7, or 4 to 7, or 5 to 7. Preferably in these ranges the upper boundary is 6 or 5. Preferably the acidic groups are all carboxylic acid groups.

The deprotonation of the acidic groups at alkaline pH may result in the aqueous solubility of the polymer being higher at the alkaline pH of 7 to 12 than at the acidic pH of less than 7. The enhanced aqueous solubility may result in a greater degree of swelling of the polymer with solvent (water) molecules, e.g. relative to the degree of swelling at the lower pH. The swelling of the polymer is in turn accompanied by formation of a gel-like composition which has an increased viscosity.

Suitable acidic groups may be sulphonic acid groups, phosphoric acid groups and carboxylic acid groups. A polymer may have a single type or a combination of two or more of such types of groups. Preferably all groups are carboxylic acid groups that have the same or different pKa. For example, carboxylic acid groups attached to saturated carbon spacers have pKa different than those attached to aromatic spacer. Such groups may for example be part of polymers disclosed herein after.

The pH induced thickener may comprise an alkali soluble emulsion (ASE) polymer.

An ASE polymer is capable of forming an alkali-soluble emulsion when dispersed in an aqueous composition at a pH below 7. Without wanting to be bound by theory it is believed that the thickening property of the ASE polymers is based on a non-associative mechanism based on polar repulsive interactions (e.g. charge-charge interactions) between polar groups occurring upon subjecting it to increasing pH values such as above 7.

One group of ASE polymers comprises the aforementioned acidic groups for example in the form of the carboxylic acid groups to provide the polar groups. Such ASE polymers may form solutions or emulsions at acidic pH due to reduced deprotonation of the acidic groups. The solutions or emulsions may have a relatively low viscosity, e.g. lower than 1500 mPa·s. Increasing the pH results in the aforementioned deprotonation and therewith formation of charged groups (conjugated base of the acid group) and concomitant increase of solubility of the polymer which is accompanied by a degree of swelling far greater than that at the acidic pH. This greater degree of swelling causes the increase in the viscosity to values higher than 5000 mPa·s or even higher values as disclosed herein.

A group of polymers having acidic groups and that may provide good ASE polymers are acrylic co-polymers comprising or consisting of monomer residues of at least one acrylic acid and at least one acrylic acid ester (acrylate residue) having an alcohol residue with less than 6 carbon atoms. Such acrylic co-polymers may have a structure as in Formula 1 in which x represents the fraction of the acrylic acid monomer residues and y represents the fraction of acrylic acid ester monomer residues. The different monomer residues may be randomly distributed along the polymer chain or not.

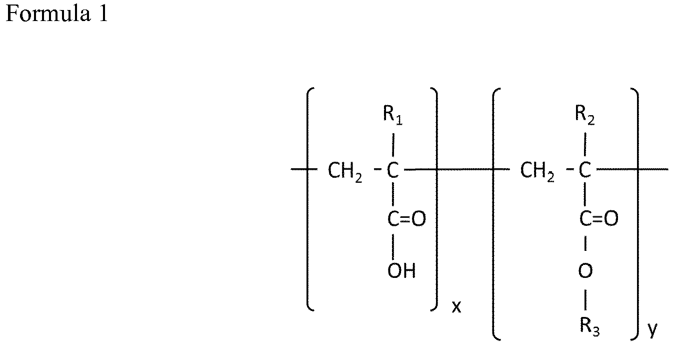

The sum of fraction x and y together may be less than 0.9, or less than 0.8 so that there may be other monomer residues, for example for crosslinking purposes. If there are no other monomer residues present the sum of fraction x and y is 1. A fraction of the carboxylic acid groups may be reacted with pentaerytritol or other di, tri, or tetra alcohol for crosslinking of polymer chains. In such case the sum of x and y may be still 1 while having crosslinked polymers.

The fractions x and y can be chosen to tailor thickening properties of the polymer. Thus x and y may be equally large. Alternatively x may be smaller than y or vice versa, where up to some maximum value, a higher x may increase the solubility upon increasing pH.

A part, or all, of each of the acrylic acid and/or the acrylic acid ester monomer residues may be substituted with Fluorine and/or alkyl groups at their carbonyl α-carbon atoms. Thus, R₁ and R₂ may each be individually chosen to be H, F, or linear or branched alkyl or alkoxy groups of less than 6 carbon atoms, preferably less than 4 carbon atoms. The alkyl or alkoxy groups can have one or more ether groups therein. Ether groups are generally considered as apolar. Preferably, the alkyl groups are methyl and/or ethyl and/or the alkoxy groups are methoxy and/or ethoxy groups. Most preferably the R₁ and R₂ are methyl and/or ethyl groups.

Thus, for example, a fraction x of acrylic acid monomer residues may comprise a first sub-fraction of monomer residues with R₁=H and a second sub-fraction of monomer residues with R₁=methyl. Likewise, a fraction y of acrylic acid ester monomer residues may comprise a first sub-fraction of residues with R₂=H and a second sub-fraction of residues with R₁=methyl.

The alcohol residue R₃ has less than 6 carbon atoms and preferably even less than 5, or 4, or even 3 carbon atoms. R₃ may be aryl, alkyl, cycloalkyl or a combination of these and may comprise ether moieties, but this is not preferred. Preferably R₃ is one or more of methyl, ethyl, propyl, butyl and/or pentyl, linear or branched where possible.

Examples of suitable acrylic ASE co-polymers are the commercially available family of polymers sold under the tradename Carbopol®. Other comparable acrylic co-polymers sold under other tradenames exist. Carbopol® 940 NF polymer may, for instance, be used. The pH of a 0.5 to 1 wt. % Carbopol® 940 NF aqueous solution or dispersion is in the range of 2.5 to 5, with a 1 wt. % solution or dispersion having a pH in the range of 2.5 to 3.0. At such pH, the viscosity of a 1 wt. % polymer solution/dispersion is well below 1500 mPa·s. Already for the 0.5 wt. % polymer variant the viscosity increases to 40,000 - 60,000 mPa·s at a pH in the range of 7.5 to 10.0. At pH of around 7, a 1 wt. % gum-like solution of Carbopol® 940 NF polymer takes the form of a stiff gel. Such Carbopol® 940 NF and other suitable Carbopol® polymers may, for example, be included in the peroxide composition at a content in the range of 0.5 wt.% to 5.0 wt.%, preferably 0.5 to 5.0 wt.% to 2.0 to 5.0 wt.%. The polymer content of the system is thus chosen such that after combination of the components of the system the resulting peroxide composition has the desired viscosity while in the first or third composition of the system the polymer does not cause a viscosity for example exceeding the desired maximum viscosity of 1500 mPa·s, 1000 mPa·s, or even 500 mPa·s.

Other suitable acrylic polymers can be used such as for example the commercially available polymers sold under the tradenames ACUSOL™ 810A, ACUSOL™ 830, ACUSOL™ 835, ACUSOL™ 842. And yet other ASE type polymers of other vendors may also be used.

Additionally or alternatively the systems pH induced thickener can comprise a hydrophobically modified alkali-soluble emulsion (HASE) polymer. A HASE polymer is capable of forming a hydrophobically-modified alkali-soluble emulsion when dispersed in an aqueous composition at a pH below 7. HASE polymers may be preferred over ASE polymers as they may provide higher viscosity gels, often in less time, while providing lower viscosity solutions and/or suspensions (such as with viscosity lower than 100 mPa·s when in acidic environment) at similar concentration levels as ASE polymers. Compared to ASE polymers, HASE polymers in general have additional relatively large hydrophobic groups. Without wanting to be bound by theory, the improved thickening effects compared to ASE polymers are believed to be due to the HASE polymers' capability to not only provide thickening through the non-associative mechanism based on polar interactions similar to those occurring with ASE polymers, but also through an associative mechanism based on apolar interactions of the large hydrophobic groups between themselves and between them and other apolar constituents of a composition they are part of (such as for example solvents, dispersion media, surfactants, dispersants etc.).

One group of HASE polymers comprises the aforementioned acidic groups in the form of for example the carboxylic acid groups. Such polymers may form emulsions at acidic pH below 7 due to their reduced deprotonation and hydrophobic modification. The emulsions may have a relatively low viscosity, often lower than for the ASE polymers described herein above. For example the viscosities may be below 500 mPa·s or even below 30 mPa·s. Adding base to such emulsion results in the aforementioned deprotonation of acidic groups and concomitant increase of solubility of the polymer which is accompanied by a degree of swelling of the polymer far greater 5000 mPa·s. For these polymers, the hydrophobic groups are believed to be freed upon dissolution so that they may participate in forming a hydrophobic group interaction based network due to the intramolecular and intermolecular hydrophobic group interactions between such hydrophobic groups and with other hydrophobic entities/molecules in a composition such as surfactants, particles, emulsion droplets, fragrants, flavorants, dyes etc. As a result of this associative mechanism of thickening, which ASE polymers typically are not capable of having, in general a more viscous gel composition results with HASE polymers than with the ASE polymers.

A group of polymers having acidic groups and that may provide good HASE polymers are acrylic co-polymers comprising or consisting of monomer residues of at least one acrylic acid and at least one acrylic acid ester (acrylate residue) having a hydrophobic alcohol residue with 6 or more carbon atoms. Such acrylic co-polymers may have a structure as in Formula 2 in which x represents the fraction of the acrylic acid monomer residues and y represents the fraction of acrylic acid ester monomer residues as defined for the ASE polymers of Formula 1 herein above and z represent the fraction of acrylic acid ester residues having a hydrophobic alcohol residue with 6 or more carbon atoms. Thus, for a HASE polymer, z may not be zero, while y may be zero. The different monomer residues may be randomly distributed along the polymer chain or not.

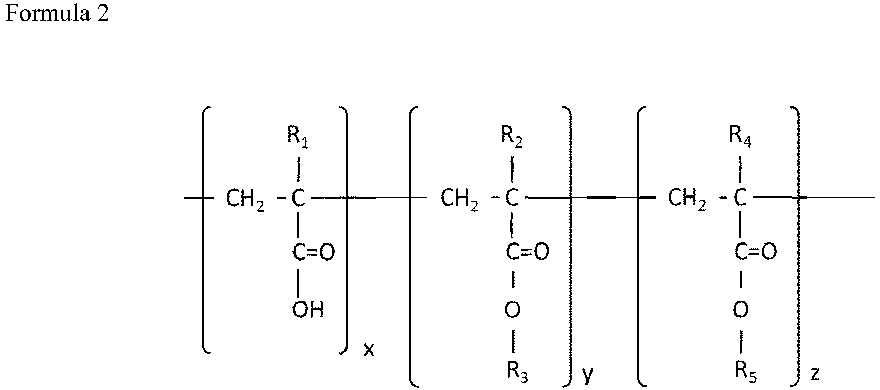

The sum of fraction x, y and z together may be 1 so that there are no other monomer residues present. Alternatively, this sum may be less than 1, such as 0.9, or less than 0.8 so that there may be other monomer residues, for example for crosslinking purposes. A fraction of the carboxylic acid groups may be reacted with pentaerytritol or other di, tri, or tetra alcohol for crosslinking of polymer chains. In such case the sum of x, y and z may be still 1 while having crosslinked polymers.

The fractions x, y and z can be chosen to tailor thickening properties of the polymer. The fractions may all be equally large. Alternatively z may be smaller than x and y or vice versa. Like with the ASE polymers, a higher x in the HASE polymers may increase solubility upon increasing the pH. As indicated herein before, z may not be 0 while y may be 0. Typically however y is also not 0.

Like with the ASE polymers, a part, or all, of each of the acrylic acid and/or the acrylic acid ester monomer residues in Formula 2 may be substituted with Fluorine and/or alkyl groups at their carbonyl α-carbon atoms. Thus, for such monomer residues R₁, R₂ and R₄, may each be individually chosen to be H, F, or linear or branched alkyl or alkoxy groups of less than 6 carbon atoms, preferably less than 5 carbon atoms and more preferably less than 4 carbon atoms. The alkyl or alkoxy groups can have an ether group therein. Preferably, the alkyl groups are methyl and/or ethyl and/or the alkoxy groups are methoxy and/or ethoxy groups. Most preferably the R₁, R₂ and R₄ are chosen from methyl and/or ethyl groups. Thus, in an example, the monomer residue fraction x, and one or more of the fractions y and z, each may comprise a first sub-fraction of monomer residues with R₁=H and a second sub-fraction of monomer residues with R₁=methyl.

The alcohol residue R₃ may be chosen as defined for Formula 1.

The alcohol residue R₅ has 6 or more carbon atoms, preferably 10 or more or even 15 or more carbon atoms. R₅ may be a C₁₀-C₄₀, or C₁₅-C₄₀ or C₁₅-C₃₀ or C₂₀-C₄₀ or C₂₀-C₃₀ group of the type linear or branched alkyl, cycloalkyl, aryl or a combination of these. R₅ may comprise ether moieties, but this is not preferred. In an embodiment the R₅ group is an alkyl or cycloalkyl group.

A particularly desired group of HASE polymers has the Formula 3 and defines a sub-groups of polymers of structure of Formula 2. In this case the fraction x of Formula 2 is given by the total of x1 and x2 in Formula 3. Rx groups may be chosen similar to those for Formula 2 with the following specifications. The fraction y is not 0. Part of the monomer residues of fractions y and z have R₄ and R₆ respectively as H.

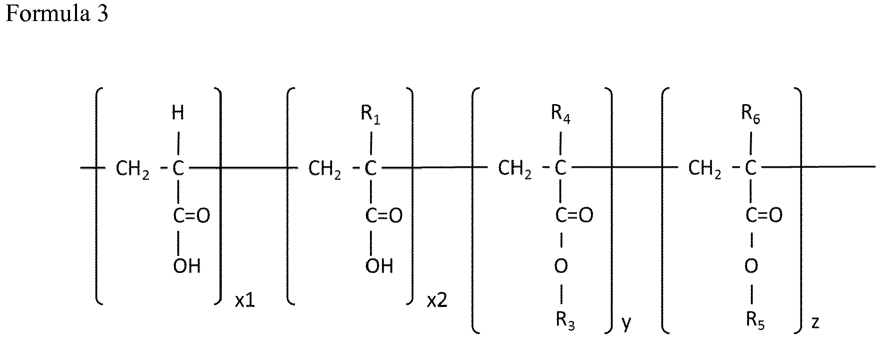

Typically x1, x2, y and z together are all non-zero and their sum is 1, but alternatively their sum may be 0.9 or 0.8 to allow for the presence of other monomers.

Examples of acrylic co-polymer type HASE polymers are commercially available as the polymers sold under the tradenames Acusol™. Others, sold by other vendors exist and can be used as well. The Acusol™ 820 forms an extremely viscous gel with a viscosity > 1.0 x·10⁵ mPa·s at pH in the range of 7.5 to 12.5 at a ∼2 wt. % active polymer content in such gel. By contrast, a very low viscosity of less than 50 mPa·s or even less than 30 mPa·s, may be observed for these polymers at pH 3.0 at contents as high as 30 wt. % in an acidic composition of a system or kit for preparation of such gel. The content of Acusol™ 820, 823 and/or other HASE polymers in the peroxide composition may be, for instance, in the range of 0.5 to 20 wt. %. A preferred range is 1 to 10 wt. %, and a more preferred range is 1 to 6 wt. %. Other content ranges of HASE polymers as the ones mentioned above in a peroxide composition may, for example, be in the range of 0.5 wt. % to 20.0 wt. %, depending on the viscosity behavior of the polymer. Preferably this content is in the range of 0.5 wt. % to 20.0 wt. % or in the range of 0.5 wt. % to 8.0 wt. %, or between 0.5 wt. % to 5.0 wt. %, or 0.5 to 3.0 wt. %. The lower content boundary in these ranges preferably is 1.0 wt. %.

As an example of how the HASE polymers function, consider combining equal amounts of an aqueous composition with 6.0 wt. % ACUSOL 820 and an aqueous composition of 0.74 wt. % sodium hydroxide, which results in a clear foam-free gel with a viscosity of >100,000 mPa·s while only containing a 0.9 wt. % ACUSOL 820's active polymer solids. Note the dilution factor due to combination of equal amounts of ACUSOL and hydroxide compositions.

All of the pH induced thickeners such as the aforementioned ASE and HASE polymers preferably do not include chemical groups or entities susceptible to oxidation by the peroxide within 15 minutes or less. Indeed such is typically the case with the specific groups of ASE and HASE polymers described herein above. In general polymers with saturated bond backbones and acidic groups will have relatively good resistance to the oxidizing agents even at high contents. The HASE type polymers, such as those described herein before have an increased salt or electrolyte content tolerance when compared to the ASE polymers, i.e. their viscosity decrease with increased electrolyte content is less. This is advantageous for high ionic content compositions (peroxide being an ionic species in free form) to be used for oral application. After all, the oral application systems may benefit from certain specific additives in salt or electrolyte form such as the remineralization and desensitizing agents as will become apparent hereinafter. HASE polymers are also more compatible with surfactants and fragrances which is useful for oral application systems too.

Another group of pH induced thickener comprises or consists of a hydroxyalkyl cellulose such as for example, hydroxyethyl cellulose or hydroxypropyl cellulose, although these compounds may be less stable against the oxidative peroxide, they are uncharged when brought to the composition pH of 7 to 12. This may have advantages compared to the acid group comprising polymers described herein before. As hydroxyethylcellulose powders e.g. Natrosol™ 250 (Ashland®) and CELLOSIZE™ QP 100MH (DOW®) have been found to be effective. These may also be dispersed and/or dissolved in suitable solvents and dispersants such as water alcohol mixtures.

The peroxide composition is a thickened or viscous composition. It preferably is a gel such as a hydrogel. The peroxide composition preferably has a viscosity that is at least higher than one or more of the system compositions and most preferably higher than all of the kit compositions (not counting the compositions in dry form). Preferably the peroxide composition has a viscosity of at least 1500 mPa·s. More preferably such viscosity is higher than any one of the following values in mPa·s: 2000, 5000, 10,000, 20,000, 50,000, 75,000, 100,000, 200,000, 500,000, 1,000,000.

Upon mixing of the systems components, the thickening may occur at a certain rate of increase of viscosity. For a pH induced thickener at standard conditions, thickening period may be taken as the time period between the moments of mixing of the components of a system and of reaching the minimum viscosity specified for the peroxide composition. The shorter the thickening period, the faster the viscosity increase, i.e. higher the rate. The thickening period is, for example, less than any one of the following maximum values in minutes: 5, 2, 1, and 0.5. Preferably it is less than 10 seconds. Preferably such period is achieved for minimum viscosities of 5000 mPa·s, 50,000 mPa·s or even 500,000 mPa·s. The period may be tailored by the type of pH induced thickeners used, HASE polymers in general providing a shorter period than ASE polymers) due to their content (HASE polymers in general providing a shorter period than ASE polymers at same or even lower content) and the pH change invoked. For example, as described herein before, the HASE type polymers may thicken faster than ASE type polymers under similar pH conditions.

The peroxide composition to be obtained with the system has a pH in the range of 7 to 12. When the kit is intended to be used on a living human or animal subject, e.g. for bodily disinfection or oral disinfection, or tooth whitening (whitening of dental pieces), a more body-compatible pH in the range of 7 to 9.5, or more preferably in the range of 8 to 9 is used. Otherwise, a peroxide composition having a pH in the range of 8 to 12, more preferably in the range of 8 to 10 can be used.

Such pH in general destabilizes, or in other words, activates the peroxide agent. This is for example useful for hydrogen peroxide in free or complexed form (e.g. carbamide peroxide). Thus, with the system, the peroxide agent is stored at acidic pH therewith reducing or minimizing its degradation and extending shelf-life, the activity of the peroxide agent as a whitening or sanitization agent is yet enhanced by the pH of peroxide composition being adjusted to the alkaline values prior to use.

Furthermore, the alkaline pH in the peroxide composition directly promotes increase of its viscosity for example compared to the viscosity of any of the first and second compositions. The more viscous or thickened peroxide composition may make application to a surface much easier.

A system at least has two compositions in which all of the ingredients of a peroxide composition are contained. This is the simplest variant of the system. There may be more compositions in a system such as for example three, four or five compositions. These may be used to distribute ingredients over according to need.

Furthermore, preferably all of the compositions comprise a solvent and/or dispersion medium such as e.g. water. In such case, there may be preferred mixing ratios of quantities of the respective compositions. Such ratios define dilution factors for ingredients of the respective compositions as upon preparation of a peroxide composition mixing of compositions occurs.

The first ratio of first quantity to second quantity preferably is chosen such that dilution factors are in the range of 0.1 to 10. A suitable first ratio of first quantity of first composition to the second quantity of second composition in such case is in the range of 0.1 to 10, or 1 to 5 or 2 to 3 or 3 to 4. For example the ratio may be chosen from the group of ratios consisting of 10, 9, 8, 7, 6, 5, 4, 3, 2, 1, 0.8, 0.7, 0.6, 0.5, 0.4, 0.3, 0.2, 0.1. Such ratios may for example conveniently be employed for two-component systems. However, they may also be chosen for a three component system.

For a three component system having a third quantity of a third component, there may be defined second ratio of first quantity to third quantity. Its values may be defined as those of the first ratio and may the same or different. Thus for example, the second ratio may also be in the range of t to 10 preferably 1 to 5. Preferably the ratios are the same, such as both 1.

One reason for having a third composition may be that certain ingredients that are not very well compatible when together for extended periods of time may be provided in separate system compositions. For example certain pH induced thickeners may slowly degrade when in contact with the strongly oxidizing peroxide agent, especially when the peroxide content is high.

Furthermore, whenever a dilution occurs of an ingredient due to combination of kit compositions, such dilution effect may be reduced by loading multiple of the compositions with an ingredient.

Two preferred system configurations consist of either two compositions or three compositions. Table 1 lists such configurations with regard to presence of the peroxide agent and the pH induced thickener. Other ingredients may be present, but these are not shown.

**Table 1**

| Kit-config. | Composition | Peroxide agent | pH induced thickener | Basic agent | 1^{st} ratio range | 2^{nd} ratio range |
|---|---|---|---|---|---|---|
| 2 compositions | 1^{st} aqueous | yes | yes | no | 10 - 0.1 | n.a |
| | 2^{nd} | no | no | yes | | |
| 3 compositions | 1^{st} aqueous | Yes or no | yes or no | no | 10 - 0.1 | 10 - 0.1 |
| | 2^{nd} | no | no | yes | | |
| | 3^{rd} | yes or no | yes or no | no | | |

The ratio ranges can be narrowed using the ranges disclosed herein above, for example the first ratio and/or second ratio can be in the range of 5 to 1.

Quantities may be expressed in volumes or weights and relative quantities in the corresponding volume ratios or weights ratios. When all compositions have solvents and/or dispersion media, the relative amounts of compositions may be expressed in volumes or volume ratios. In such cases the densities of each of the compositions need to be taken into account for calculation of weight based dilutions unless such densities are substantially the same for compositions.

Depending on the ratios a dilution or thinning effect of the peroxide and/or the pH induced thickener, as well as of other ingredients will occur upon combining of the system compositions. As usual in the art, contents of ingredients of system compositions can be calculated from the contents in the peroxide composition made with the corresponding system, or vice versa, using appropriate dilution or thinning factors based on composition ratios as provided herein above.

The quantities of system compositions and/or the ratios with which they are combined during use may be predetermined so as to result in a fixed viscosity and fixed content of ingredients in the peroxide composition when the system is used.

Alternatively, the relative quantities of compositions may to some extent be user selected albeit to such extent that the final composition pH is within the required values of 7 to 12. For example, the quantity of the second composition to the total quantity of other compositions may be user selected so that a pH and/or active ingredient content may be modified by user selection. Controlling the pH in this manner may enable the user to control the properties of the peroxide composition, e.g. the peroxide compound activation and rheological behavior of the composition. The latter may be possible because a more alkaline pH may lead to a greater degree of viscosity change or thickening as described herein. A dispenser device for user selection of composition ratios will be described herein after.

The peroxide content in the first quantity of the first composition is preferably chosen such that the entire content of peroxide in the peroxide composition is within anyone of the ranges described for the peroxide composition, taking into account any dilution upon combination of compositions and ingredients of a system to arrive at the peroxide composition. For a system in which only a single composition includes the peroxide agent the entire content of peroxide is contained in this single composition being the first composition. For a 3 or more composition system in which more than one compositions include the peroxide agent, the entirety of the combined peroxide content in a peroxide composition is thus distributed over the more than one system compositions. For all of these configurations the actual content of the peroxide agent in a particular composition can be calculated from the combined content using the dilution factors as indicated herein above and the weight of that composition in the usual way.

Preferably the content of peroxide agent in a single system composition such as the first composition does not exceed 0.0176 M_{pg}/g (∼60 wt. % H₂O₂) based on the weight of the single system composition, where M_{pg}/g defines a molality in terms of moles of peroxide groups per weight of composition.

The peroxide agent content in a composition such as the first aqueous composition or the third composition is preferably lower than any one of the following values: 0.0147 M_{pg}/g (50 wt. % H₂O₂), 0.0132 M_{pg}/g (45 wt. % H₂O₂), 0.0118 M_{pg}/g (40 wt. % H₂O₂), 0.00882 M_{pg}/g (30 wt. % H₂O₂), 0.00588 M_{pg}/g (20 wt. % H₂O₂), 0.00294 M_{pg}/g and (10 wt. % H₂O₂). The content can be in the range of 0.00588 M_{pg}/g (20 wt. % H₂O₂) and 0.000882 M_{pg}/g (3.0 wt. % H₂O₂) or in the range of 0.00294 M_{pg}/g (10 wt. % H₂O₂) and 0.00147 M_{pg}/g (5.0 wt. % H₂O₂). This may be e.g. suitable for home use tooth whitening purposes. Contents higher than 0.00294 M_{pg}/g (10 wt. % H₂O₂) are useful for chairside (at the dental professional) whitening purposes. These contents can be in the range of 0.00294 M_{pg}/g (10 wt. % H₂O₂) and 0.0118 M_{pg}/g (40 wt. % H₂O₂) or in the range of 0.00294 M_{pg}/g (10 wt. % H₂O₂) and 0.00588 M_{pg}/g (20 wt. % H₂O₂), or in the range of 0.00588 M_{pg}/g (20 wt. % H₂O₂) to 0.0118 M_{pg}/g (40 wt. % H₂O₂). Such higher contents give shorter treatment times and/or better whitening effect, but are also more dangerous in use. Peroxide contents lower than any of the following values 0.00147 M_{pg}/g (5 wt. % H₂O₂), 0.000588 M_{pg}/g (2 wt. % H₂O₂), or 0.000294 M_{pg}/g (1 wt. % H₂O₂) may be appropriate when the system is to be applied, for instance, in a fresh instant whitening toothpaste application. Corresponding range values in wt. % carbamide peroxide or any other peroxide or a mixture of compounds can be calculated as indicated herein above from the peroxide agent contents in M_{pg}/g. In the above ranges, the contents refer to amounts of peroxide agent per weight unit of peroxide composition, regardless of whether such amounts of peroxide agent are in dissolved and/or dispersed state. It will be clear that for a composition where a dissolved peroxide agent is in a dissolved state, a range is maximized by the maximum solubility of that agent in the relevant solvent.

The peroxide content in the first aqueous composition and a third composition may be the same or different.

The combined content of the pH induced thickener present in all compositions of a system as a percentage of the total weight or volume of all compositions in a system including the compositions not having a pH induced thickener content such as the second composition, may be selected as described herein before for the pH induced thickener in the peroxide composition.

The amount of pH induced thickener is preferably in the range of 1 to 50 wt. %, more preferably in the range of 1 to 30 wt. % and most preferably in the range of 5 to 25 wt. % in the system compositions. These ranges may combine well with the suitable dilution factors and mixing ratios provided herein above. Thus the combined content may be in the range of 0.5 to 20 wt. %, more preferably in the range of 0.5 to 10 wt. % or 2 to 10 wt. % of the composition of the system. %. The contents may be present in only the first composition and/or the third composition.

The first aqueous composition needs a first pH less than 7 to stabilize the peroxide agent and if present also a pH induced thickener. For example, hydrogen peroxide and its complexes such as carbamide peroxide are more stable at pH lower than 7 than at pH higher than 7. Preferably, the first pH is less than any one of the following values: 6, 5, 4, 3, 2. Preferably it is less than 5 or 4. The first pH may be in the range of 2 to 5, such as in the range of 2 to 4. Hydrogen peroxide, for example, may be particularly stable below pH 4. The first pH reduces peroxide agent degradation and may even obviate the need for a peroxide stabilizer to be added. The system therewith has an increased shelf life and high active peroxide content even after prolonged storage. The first pH also reduces, minimizes or prevents the pH induced thickener to cause substantial thickening of the first aqueous composition.

Whenever a third composition is present in the system, such as a third aqueous composition, and this composition comprises a peroxide agent and/or a pH induced thickener, then such composition preferably has a third pH similar to the first pH. The third pH may be equal to the first pH, but this need not be so.

The First pH and third pH may require adjustment to a desired value after having added any peroxide agent or pH induced thickener to it. Such adjustment may be done using appropriate acids and/or bases. Strong acids may be used such as phosphoric acid and weak acids may be used such as acetic acid or citric acid or other. However, for some of the acidic polymers disclosed herein (e.g. the acrylic copolymers of the ASE or HASE type) the adjustment of first or third pH may be lessened or not needed at all due to the intrinsic acidity of the polymers.

The basic agent is part of the second composition. The second composition may have a second pH higher than 7 for example when it includes water. The basic agent may comprise or consist of one or more bases or basic compounds capable of releasing or causing the release of hydroxide ions (OH⁻) in an aqueous environment. Each one of such base may be a Bronsted or Arrhenius base and/or may be a weak or strong base.

Suitable strong bases include but are not limited to hydroxides of alkali metals and alkali earth metals as well as of ammonium based cations. Particularly suitable are NaOH, KOH, Ça(OH)₂ , Sr(OH)₂ and NH₄OH. Such bases may enable facile and low toxicity adjustment of the pH of the composition which is useful for oral application of the system such as with whitening. All of these with the exception of Sr(OH)₂ is readily soluble in water at standard conditions.

The metal oxide precursors of the above alkali metal and alkali earth metal hydroxides may also be used for example as solid state basic agent. Such oxides will generally be converted to the hydroxides upon contact with water, i.e. upon use of the system.

Suitable weak bases include but are not limited to salts of conjugated bases of weak acids. These may include for example the salts of conjugated bases of carboxylic acids and/or phosphoric acids. Example carboxylic acids salts comprise carbonates, bicarbonates, acetates, citrates. Salts of conjugated acids of phosphoric acid include the di-, tri-cationphospates. However, other examples include salts of sulphates, (divalent salt) borates and silicates.

The second composition may comprises a solvent and/or a dispersion medium in which the basic agent is dissolved and/or dispersed. Preferably the second composition is an aqueous composition. More preferably the second composition is a solution of the basic agent with a second pH higher than 7. When dispersed, suitable dispersants and/or surfactants may be present.

The second composition may be solvent-free and/or dispersion medium free. Since many of the bases mentioned herein before generally are solids at standard conditions, a basic agent having one or more of such compounds will often be a solid. This dry second composition is useful when a dilution of constituents of the systems ingredients, such as e.g. the peroxide agent, due to combination of the compositions of the system upon use is to be reduced or prevented.

The amount of basic agent is chosen to be sufficient to compensate any acidic entities in the final peroxide composition to the extent that the composition pH is in the required range of 7 to 12. The second composition may be provided with such an amount of basic agent that the final pH of the peroxide composition may be tuned by altering the relative quantities of second composition to first aqueous composition and/or third composition (i.e. the first and second volume or weight ratios) as described herein before. The hydroxide content in the second composition may be in the range of 0.001 to 1.0 M, preferably in the range of 0.01 to 1.0 M or in the range of 0.1 to 1.0 M. In an example a range of 0.01 to 0.05 M is used. In a preferred embodiment, the second composition comprises 0.01 to 1.0 M of potassium hydroxide, sodium hydroxide or ammonium hydroxide.

The first aqueous composition may have a first viscosity and a third composition having a solvent and/or dispersion medium may have a third viscosity. These viscosities are lower than the final peroxide gel composition viscosity. The viscosity of such compositions preferably are independently chosen to be below 2000 mPa·s, more preferably below 1500 mPa·s or below 1000 mPa·s n and even more preferably below a 500 or 100 mPa·s. Such low viscosity values and especially values below 500 mPa·s provides improved flowability of compositions and therewith easier handling during manufacture and use. In some embodiments, for example using the acrylic co-polymer HASE type pH induced thickeners, the viscosity of the first aqueous composition including the pH induced thickener can have a viscosity of less than 50 mPa·s.

The induced change of viscosity of systems compositions to the peroxide composition upon use of the system may occur rapidly due to the use of the pH induced thickeners. For a pH induced thickener at standard conditions, the time period for thickening to an aforementioned minimum viscosity in the peroxide composition counted as of system composition combination is, for example, less than any one of the following maximum values in minutes: 5, 2, 1, and 0.5. Preferably it is less than 10 seconds or even virtually instantaneous. Instantaneous may mean directly upon leaving of a peroxide composition from the tip of a mixing syringe The rate of viscosity increase of the peroxide composition may dependent on the type of pH induced thickeners used, their content and the pH change invoked. For example, as described herein before, the HASE type polymers may thicken faster than ASE type polymers under similar pH conditions. Without wanting to be bound by theory, it is believed that HASE polymers have the additional associative thickening mechanism as compared to the ASE polymers and therefore may thicken faster.

In an embodiment, the system is for preparing a peroxide composition for oral use such as for oral disinfection and/or tooth whitening. In such case the system optionally but preferably comprises one or more additives selected from the group consisting of: a tooth desensitizing agent; a tooth remineralizing agent; and a coloring agent and a flavoring agent.

Any one or more of these additives may be added to the first aqueous composition and/or second composition and/or third composition if present in the system provided that such additive is to at least some extent chemically compatible with chemical conditions (e.g. pH or oxidising properties) within such compositions. Alternatively, when chemical compatibility is reduced and for example slow degradation occurs, the additive may be added to the third composition or a further fourth or fifth composition not having such degrading properties. For example, such compositions may have a neutral pH or lack presence of the peroxide agent.

An additive may be entirely present in one single system composition. Alternatively, since some of the additives comprise subparts such as the different ions of additives in the form of salts, the different subparts of an additive may be distributed over two or more system compositions as will become apparent herein after.

In an embodiment the desensitizing agent comprises or consist of potassium ions or one or more sources of potassium ions such as potassium salts. The potassium ions are believed to desensitize nerve fibers blocking the neural transmission. The one or more potassium salts may be chosen from the group consisting of: potassium chloride, potassium fluoride, potassium citrate, potassium nitrate, potassium carbonate and potassium hydroxide. Other potassium salts can be used. The desensitizing agent or parts of it may be present in two or more compositions. Thus, the second composition can have for example potassium hydroxide while the first composition includes potassium chloride or potassium nitrate. Alternatively, the potassium ions can be part of the first component e.g. as potassium chloride and nitrate ions, while counterions such as hydroxide, chloride and/or nitrate ions are part of the second composition with cations different from potassium, such as e.g. sodium or ammonium ions.

The content of potassium ions and counter ions should be controlled in their related compositions so that they will produce no more potassium than the equivalent of 5 wt. % potassium nitrate in the peroxide composition to meet regulations requirements. The preferred range of potassium ions in the compositions of a system amounts to the equivalent present in 1.0 wt. % - 4.5 wt. % of potassium nitrate in the peroxide composition. Again dilution factors and mixing ratios as described herein before may need to be taken into account.

In an embodiment the desensitizing agent or remineralization agent comprises or consists of one or more compounds also suitable for plugging dental tubules. For example, such compounds may be any one of: Sodium fluoride, Stannous fluoride, Strontium chloride, Silver diamine fluoride, Potassium oxalate, Calcium phosphate, Calcium carbonate. Preferably the system comprises a calcium ions or a source of calcium ions and/or strontium ions and monobasic, and/or dibasic and/or tribasic phosphoric acid ions (phosphoric ions) or a source of any one or more of these. Preferably the calcium and/or strontium ions or their sources are comprised within an acidic composition such as the first composition and/or third composition if the latter is acidic e.g. in soluble salt form such as e.g. calcium nitrate and/or strontium nitrate. Such compounds need the acidic environment if they are required to be in solution. The phosphoric ions and their sources are preferably comprised within an alkaline environment such as in the second composition and/or the third composition if this one is alkaline. The separated sources (precursors for the desensitizer and/or remineralizing agent) system allows convenient storage, while it provides in situ formation of calcium phosphate (CP) and/or strontium phosphate (SP) in crystalline or, more preferably amorphous form (ACP and ASP) in the peroxide composition upon mixing of the compositions when the system is used.

Generally, the content of calcium ions and the content of phosphoric ions or carbonate ions in the system compositions should be dosed within the range that can produce 0.1 wt. % to 1.0 wt. % CP or ACP, respectively, (around 0.5 wt. % preferred) in the finished peroxide composition.

Detailed materials and contents of additives such as ACP or potassium nitrate that can be used in the current systems and how they are to be distributed over multiple compositions of a system are disclosed in US9138386B2, which is incorporated by reference in its entirety.

In an embodiment the desensitizing agent comprises or consists of the one or more compounds for plugging dental tubules and potassium ions and/or a source of potassium ions.

Suitable surfactants for the peroxide composition or any one of the system compositions include polyol surfactants, such as Pluronic F-127. Other surfactants, such as Cremophor RH-40, USP and Crodateric CAB 30-LQ, may also be employed.
The peroxide composition and the system may further comprise a flavoring agent. The flavoring agent may be present in one or more compositions of the system. The flavoring agent may, for instance, be included in the first composition, and/or the second composition. Alternatively it may be present in the optional third composition. The possibility of including the flavoring agent in the composition separate from the first and/or second composition may enable more flexibility in terms of the flavor options because providing the flavoring agent separately in this manner reduces the risk that the flavoring agent is degraded by a strong oxidizing agent and/or strong acid or base. This contrasts with conventional systems which tend to have a restricted flavor choice because of the requirement to select flavoring agents according to their stability over a relatively long storage time in the presence of the other ingredients.

A suitable flavoring agent may include natural peppermint oil, which may provide the user with a sense of cleanliness and freshness, particularly when the peroxide composition is applied in treatment of oral disease which may be accompanied by the bad breath. In some non-limiting examples, sweeteners, such as xylitol, sodium saccharin, may also be used, albeit usually more sparingly as compared with other sweetened products, since excessive sweetness may be undesirable for oral products, due to the negative perception of sweetness in relation to dental health.

Example systems are provided herein below in Tables 2 and 3. In these examples all compositions are aqueous compositions. Preferably, the second aqueous composition is a solution of the basic agent. These kits can be used for whitening or sanitization purposes.

**Table 2**

| Two-composition kit or system | |
|---|---|
| First aqueous composition | • Solution or dispersion of the peroxide agent (e.g. H₂O₂ and/or carbamide peroxide) and the pH induced thickener (acrylic co-polymer of the ASE and/or HASE type as disclosed herein before); |
| | • first pH in the range of 2 to 7 and preferably of 2.0-5.0; Optionally: |
| | • at least one additive of: Ca²⁺ and/or Sr²⁺ ions (e.g. from Ca(NO₃)₂); K⁺ ions (e.g. from KNO₃) and Flavoring agent(s). |
| Second aqueous composition | • Solution or dispersion of basic agent (e.g. alkali metal hydroxide such as: NaOH and/or KOH); Optionally: |
| | • at least one additive of: solvent (e.g. ethanol and/or polyethyleneglycol); surfactant(s); K⁺ ions (e.g. from KNO₃) ; phosphate ions (e.g. from Na₂HPO₄); Flavoring agent(s) |

**Table 3**

| Three-composition kit or system | |
|---|---|
| First aqueous composition | • Solution or dispersion of the peroxide agent (e.g. H₂O₂ and/or carbamide peroxide); |
| | • first pH in the range of 2 to 7 and preferably of 2.0-5.0; Optionally: |
| | • pH induced thickener (acrylic co-polymer of the ASE and/or HASE type as disclosed herein before); |
| | • at least one additive¹ of: Ca²⁺ and/or Sr²⁺ ions (e.g. from Ca(NO₃)₂); K⁺ ions (e.g. from KNO₃) and Flavoring agent(s). |
| Second aqueous composition | • Solution or dispersion of basic agent (e.g. alkali metal hydroxide such as: NaOH and/or KOH); Optionally: |
| | • at least one additive of: solvent (e.g. ethanol and/or polyethyleneglycol); surfactant(s); K⁺ ions (e.g. from KNO₃) ; phosphate ions (e.g. from Na₂HPO₄); Flavoring agent(s) |
| Third aqueous composition | • pH induced thickener (acrylic co-polymer of the ASE and/or HASE type as disclosed herein before); |
| | • third pH in the range 2 to 7 and preferably of 2.0-5.0; Optionally: |
| | • Solution or dispersion of the peroxide agent (e.g. H₂O₂ and/or carbamide peroxide); |
| | • at least one additive, of: e.g. solvent(s); surfactant(s); K⁺ ions (e.g. from KNO₃) ; source of phosphate ions (e.g. from Na₂HPO₄); Flavoring agent(s) |

The at least one additive must be compatible with the peroxide agent and the pH values holding for a composition such additive is part of.

In the examples above, the first and/or third pH is preferably in the range of 2 to 5. In one alternative of the 3-composition system of Table 3, the first and third composition include pH induced thickener for reasons described herein before. In yet another alternative the third composition also comprises peroxide agent for reasons described herein before.

The at least one additives may be present in all of the compositions, but may also be present in only one or two of the compositions. For example if an additive is not compatible with certain compositional characteristics such as e.g. alkaline pH or acidic pH it may be added to an appropriate one or not at all. Thus, in one alternative the third composition has a third pH of 7 or around 7 and dos not include the peroxide agent such that any additives such as flavoring agents are stable in such third composition. This may be advantageous for sweeteners such as the non-nutritious ones sucralose or sodium sacharine.

In one example there may be more compositions than three. For example the flavoring agent may be separated from the first, second and third compositions in a fourth composition. This composition may have a pH of 7. Alternatively, such fourth composition may lack any solvent and/or dispersion medium. If an additive is not compatible with either basic or acidic conditions it may be separated from such basic or acidic compositions in such fourth composition. A further fifth composition may be present having all of the at least one additives while the other compositions have none of these additives. System 100 having more than five compositions are also conceivable. Providing the various ingredients separately may benefit the storage stability of the ingredients.

There may be a trade-off with the number of compositions in a system and the convenience of preparing the peroxide composition with such system. The two- and three-composition system are most preferred when it comes to convenience. When several separate compartments are required to contain the respective components, the complexity of the design of the dosing system may also increase.

A number of specific examples of two-component kits or systems, is provided as System 1 and System 2 in Table 4. Wt. % values of an ingredient are with respect to total weight of the respective first and/or second composition the ingredient is part of. The kits of Table 4 can be used for whitening or sanitization purposes.

**Table 4**

| **First aqueous composition** | | | |
|---|---|---|---|
| **System 1** | | **System 2** | |
| Ingredient | wt.% | Ingredient | wt.% |
| H₂O | 80.50 | H₂O | 49.00 |
| H₂O₂ | 15.00 | H₂O₂ | 40.00 |
| Carbopol® 940 NF | 2.50 | Acusol™ 820 | 9.00 |
| KNO₃ | 2.00 | KNO₃ | 2.00 |
| | | | |

| **Second aqueous composition** | | | |
|---|---|---|---|
| **System 1** | | **System 2** | |
| Ingredient | wt.% | Ingredient | wt.% |
| H₂O | 64.85 | H₂O | 64.75 |
| KOH | 0.65 | KOH | 0.75 |
| KNO₃ | 4.00 | KNO₃ | 4.00 |
| Propylene Glycol | 16.00 | Propylene Glycol | 16.00 |
| Glycerine | 8.00 | Glycerine | 8.00 |
| Nat. Peppermint Oil PE-5523 | 1.00 | Nat. Peppermint Oil PE-5523 | 1.00 |
| Cremophor RH-40 USP | 3.00 | Cremophor RH-40 USP | 3.00 |
| Crodateric CAB 30-LQ | 0.50 | Crodateric CAB 30-LQ | 0.50 |
| Pluoronic F-127 | 2.00 | Pluoronic F-127 | 2.00 |

For the System 1 and System 2, both compositions are aqueous compositions and their densities are approximately the same so that dilution based on volume ratio is approximately equal to weight ratio. With regard to the System 1 and System 2 the volume ratio of the first aqueous composition 102 to the second composition 104 may, for example, be 2. This would afford a final peroxide composition with 10 wt. % and 26.7 wt. % hydrogen peroxide for the System 1 and System 2, respectively. A volume ratio of 1 would results in a final peroxide contents of 7.5 wt. % and 20 wt. % for the System 1 and System 2, respectively. Other volume or weight ratios of the compositions may be used as indicated herein before.

The content of potassium hydroxide (base) may have to be adjusted on occasion in order to provide that the composition pH is in the required range of 7 to 12. The content of peroxide agent can be increased or lowered in the first aqueous composition to adjust the final peroxide content in the peroxide composition as evidenced with System 1 and System 2. Carbamide peroxide may replace H₂O₂ or be added to H₂O₂ and contents calculated as descried herein before. The pH of the first aqueous composition in Table 4 is typically in the range of 2.0 to 4.0 depending on the grade and quality of the raw ingredients hydrogen peroxide and Carbopol® 940 NF or Acusol™ 820, which both are pH induced thickeners in the form carboxylic acid group containing acrylic co-polymers. If a change of type or content of such pH induced thickeners is desired, in order to change e.g. viscosity requirements, a suitable acid may be used such as for example phosphoric acid to adjust the first aqueous composition pH.

Furthermore, while the viscosity of the aqueous compositions of both systems will typically be below 50 mPa·s (for example in the range of only 15 to 20 mPa·s), with such mixing ratio, the peroxide compositions formed will be hydrogels with high viscosities of >100,000 mPa·s for system 1 and >200,000 mPa·s for system 2. The formation of such gels will be almost instantaneous (see below).

The pH of the aqueous second component in the systems is typically in the range of 2.0 to 4.0 depending on the grade and quality of the Acusol™ 820, which is a pH induced thickener in the form of a HASE acrylic co-polymer. If a change of type or content of such pH induced thickener is desired, in order to change e.g. viscosity requirements, a suitable acid may be used such as for example phosphoric acid to adjust the first pH.

**Table 5**

| **First aqueous composition** | | | |
|---|---|---|---|
| **System 3** | | **System 4** | |
| Ingredient | wt.% | Ingredient | wt.% |
| H₂O | 73.00 | H₂O | 60.00 |
| H₂O₂ | 15.00 | H₂O₂ | 30.00 |
| Acusol™ 820 | 10.00 | Acusol™ 820 | 8.00 |
| KNO₃ | 2.00 | KNO₃ | 2.00 |
| | | | |

| **Second aqueous composition** | | | |
|---|---|---|---|
| **System 3** | | **System 4** | |
| Ingredient | wt.% | Ingredient | wt.% |
| H₂O | 64.85 | H₂O | 99.35 |
| NaOH | 0.65 | NaOH | 0.65 |

For the System 3 and System 4, both compositions are aqueous compositions and their densities are approximately the same so that dilution based on volume ratio is approximately equal to weight ratio. The volume ratio of the first aqueous composition 102 to the second composition 104 may, for example, be 1. This would afford a final peroxide composition with 7.5 wt. % and 15 wt. % hydrogen peroxide for the System 3 and System 4, respectively. The System 3 (as did the system 1 and 2) thickened instantly upon mixing of the system compositions to highly viscus peroxide compositions. Stability of gels was tested with system 4 to be at least 18 months and substantially no loss of peroxide content was observed. Other volume or weight ratios of the compositions may be used as indicated herein before.

A specific example of a 3-component system, System 5 is provided in Table 6. Wt. % values of an ingredient are with respect to total weight of the respective first and/or second composition the ingredient is part of.

**Table 7**

| **First aqueous composition** | |
|---|---|
| Ingredient | wt. % |
| H₂O | 68.00 |
| H₂O₂ | 30.00 |
| KNO₃ | 2.00 |
| | |

| **Third composition** | |
|---|---|
| Ingredient | wt. % |
| H₂O | 95.5 |
| Carbopol® 940 NF | 4.50 |
| | |

| **Second composition** | |
|---|---|
| Ingredient | wt. % |
| H₂O | 53.10 |
| KOH | 1.20 |
| KNO₃ | 10.00 |
| Propylene Glycol | 16.00 |
| Glycerine | 8.00 |
| Nat. Peppermint Oil PE-5523 | 1.80 |
| Cremophor RH-40 USP | 5.40 |
| Crodateric CAB 30-LQ | 0.90 |
| Pluoronic F-127 | 3.60 |

The pH of the third composition in System 5 is typically around pH 3.0 to 4.0.

The volume ratio of the first aqueous composition to the third aqueous composition to the second composition may, for example, be 1 to 1 to 1 to afford a final peroxide composition with 10 wt. % H₂O₂.

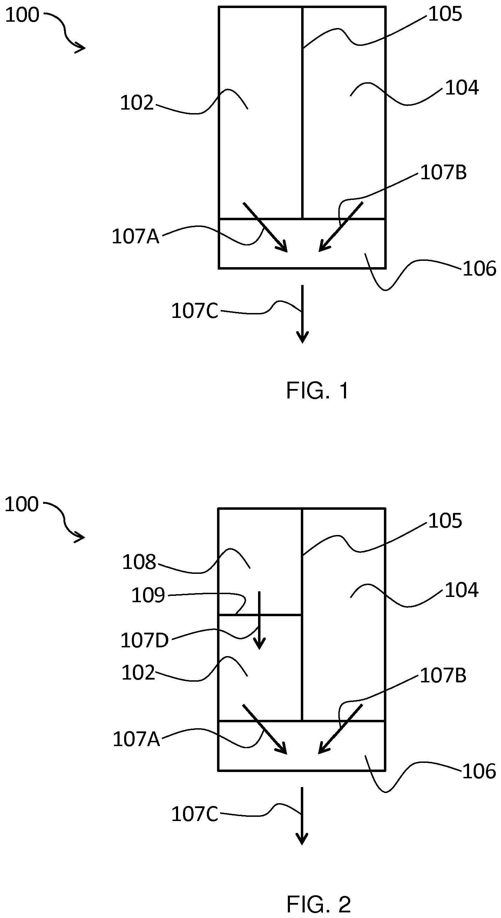

FIG. 1 depicts a kit or system 100 in which the first aqueous composition 102 is separated from the second composition 104 by a dividing wall 105 which ensures that the first aqueous composition 102 does not contact or combine with the second composition 104 prior to combining of the respective components 102, 104.

Separation of the first aqueous composition 102 and the second composition 104 may be implemented in any suitable manner. For example there may be multiple vessels or bottles or multiple compartments of a single device. The single multi-compartment device can for example comprise a multi-compartment bottle, multi-compartment syringe, or an alternative multi-compartment dispensing system capable of separately containing the compositions or ingredients of the system. The compartments may be made of any material suitable for storing the system compositions and ingredients. Particularly suitable are materials substantially resistant to the peroxide agents and/or acidic and/or basic conditions such as for example plastic (e.g. polyethylene, polypropylene, or other) or glass.

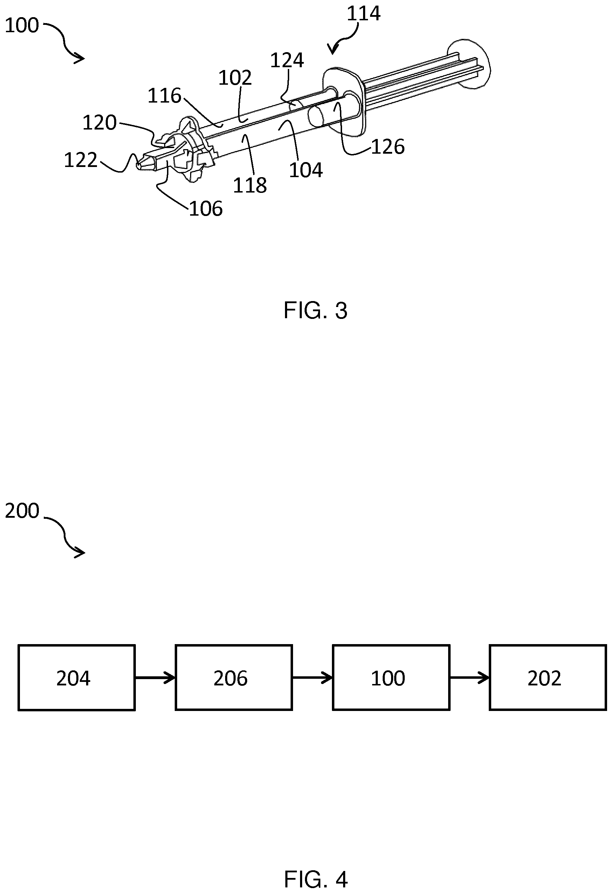

The kit or system 100 comprises (and this is optional) mixing chamber 106 (may be a simple container or compartment) in which the respective compositions 102, 104 may be combined or mixed. The arrow 107A represents the passage of the first aqueous composition 102 into the mixing chamber 106. The arrow 107B represents the passage of the second composition 104 into the mixing chamber 106. Once the respective compositions have been combined or mixed in the mixing chamber 106, the alkaline pH activates the peroxide agent and triggers the pH induced thickener to cause the increase of viscosity. The resulting mixture may then be retrieved from the chamber and used. The retrieval or passage of the composition out of the container 106 is schematically represented by the arrow 107C. The mixing chamber 106 may, for instance, be included as or in a mixing tip of a syringe, as will be explained in more detail with reference to FIG. 3. The retrieval of composition form the mixing chamber may be done directly upon mixing so that full viscosity increase may not yet have been achieved. This may be advantageous as many of the kit embodiments will eventually result in high viscosity peroxide compositions that sometimes may be difficult to manipulate or retrieve from the chamber. For example if it needs to be flow through narrow openings or chamber outlet or the like. Alternatively, there may be a waiting time to have the mixture achieve its final viscosity. Manipulation using a spatula may then be easier.

The pH induced thickener is included in the first aqueous composition 102. As described herein before, this may simplify the kit 100 relative to the scenario where the first aqueous composition 102 and the pH induced thickener are provided separately from each other.

FIG. 2 shows an example in which the pH induced thickener is provided separately from the first aqueous composition 102 and the second composition 104. In this case, the pH induced thickener is provided in a third composition 108 separated from the first aqueous composition 102 by the further dividing wall 109. Both the pH induced thickener and the first aqueous composition 102 are separated from the second composition 104 by the dividing wall 105.

The first aqueous composition 102, the second composition 104 and the third component 108 may, for example be contained in respective compartments of a tri-compartment bottle, a tri-compartment syringe, or an alternative tri-compartment dispensing system capable of separately containing the respective compositions 102, 104, 108. For example in the tri-compartment syringe there would be three compartments each having an outlet towards the mixing chamber 106 such that upon use the contents of these compartments is mixed together simultaneously.

Initial forming of a pre-combination of the first aqueous composition 102 and the pH induced thickener under acidic conditions may permit its effective, e.g. intimate, mixing with the peroxide agent, and any other ingredients contained in the first aqueous composition 102. This effective mixing may be due to the acidic conditions ensuring the pH induced thickener to cause a relatively low viscosity state of the pre-composition upon mixing. Subsequent mixing of the pre-combination with the second composition 104 may afford the higher viscosity peroxide composition, much like described for the first system.

This pre-combination is represented in FIG. 2 by the arrow 107D between the third component 108 and the first aqueous composition 102. The pre-combination may be carried out in any suitable manner, such as by manually dispensing the pH induced thickener into the first aqueous composition 102, or vice versa. More elaborate means of carrying out the pre-combining may also be contemplated, such as by providing a frangible further dividing wall 109; the user breaching the further dividing wall 109 so as to permit the first aqueous composition 102 and the third composition 108 to combine with each other.

The pre-combination composition is supplied to the mixing chamber 106, as schematically represented by the arrow 107A. The second composition 104 is also supplied to the mixing chamber 106, as represented by arrow 107B. Following combining, e.g. mixing, of the pre-combination composition and the second composition 104, the resulting peroxide composition may be retrieved from the mixing chamber much like descried for the example of Fig. 1. Again, the mixing chamber 106 may, for instance, be included as or in a mixing tip of a syringe, as will be explained in more detail with reference to FIG. 3.

The kit 100 of FIG. 3 comprises a dual-compartment syringe 114. A first compartment 116 of the syringe 114 contains a first aqueous composition 102. A second compartment 118 of the syringe 114 contains a second composition 104. Whilst a dual-barrel syringe 114 is shown in FIG. 3, a third barrel (not shown) may also be included, e.g. for the third composition 108, as previously described, but this is not shown for reasons of brevity. From the description of the kit and syringe of Figs. 2 and 3, those skilled in the art will know how to implement a triple-barrel syringe having the desired contents.

In a kit of Fig. 3 as described herein before, the third composition 108 preferably is a composition having a solvent and/or dispersion medium of a pH less than 7.

In an alternative example, the third composition 108 may be solvent-free and/or dispersion medium free; the pH induced thickener for example being included in a powder form.

The syringe 114 includes a mixing tip 120 for receiving the contents of the respective barrels 116, 118, and that of a further barrel in case a third composition is present. In this respect, the mixing tip 120 includes the mixing chamber 106. Moreover, an outlet 122 is provided in the mixing tip 120 for permitting the peroxide composition to exit the syringe 114.

In this example any two composition kit described herein can be used. Likewise, in a triple barrel syringe any of the three composition kits described herein may be used.

Such a multi-barrel syringe 114 may provide a convenient means of providing the kit 100 to the user. The respective compositions 102, 104, and optionally 108 may be combined in the mixing tip 120, i.e. upon supplying the mixing tip 120 with the respective compositions 102, 104, 108. Plungers 124, 126 may, for instance, be used to force the compositions out of the respective barrels and into the mixing tip 120. Continued forcing, e.g. via the plungers 124, 126, may cause the composition to emerge from the outlet 122, whereupon the composition may be used.

A detailed description of how such syringe, and/or an appropriate mixing tip may be designed and used is provided for example in US8581998 and US6698622 which are incorporated by reference.

Numerous alternative means of implementing the kit 100 may also be contemplated, such as using a multi-chamber, e.g. dual-chamber, bottle, or a dispensing system having a plurality of compartments. For example, the system may include two compartments which act as respective reservoirs for the first 102 and second 104 compositions; the reservoirs being filled according to the required mixing ratio of the respective compositions 102, 104.

FIG. 4 shows a block diagram of a system 200 for dispensing a peroxide composition. In the dispensing systems 200 the compartments may be discrete and separable (such as with separate bottles) from each other, or may be integrated as three reservoirs in the same device. The system may, for instance, comprise a suitable pump or vacuum arrangement for effecting simultaneous withdrawal of composition from each compartment, via separate draw pipes, and for subsequently discharging the respective compositions into a mixing chamber 106 that may be part of or separate from the system. The resulting composition just after mixing may be expelled or retrieved from the mixing chamber as described for the example of Fig. 1. The system 200 may comprise a kit 100 as described herein before and a dispensing pump 202 for dispensing the composition from the mixing chamber 106.

The kit 100 may, for example, take the form of a replaceable cartridge for the system 200, with the respective compositions 102, 104, and optionally 108 and separate parts, if present, being contained in separate compartments of the cartridge.

As shown in the block diagram of FIG. 4, the system 200 comprises a user interface 204 for enabling user selection of parameters relating to one or more of the first aqueous composition 102, the second composition 104, and the third composition 108, if present. Parameters may alternatively or additionally be selected via the user interface 204 in respect of the first separate part and/or the second separate part. The parameters may, for example, relate to which of the various components 102, 104, 108 and parts to include in the composition, and/or their respective amounts.

The system 200 shown in FIG. 4 includes a selection module 206 coupled to the user interface 204. The selection module 206 controls dosing of the respective compositions and/or the first separate part and/or the second separate part to the container/mixing chamber 106 based on the user selection. The selection module 206 may, for example, enable simultaneous withdrawal of the compositions 102, 104, 108 from their respective compartments, via separate draw pipes, and discharge the fluid to the container/mixing chamber 106.

The user interface 204 may, for instance, enable the user to select parameters which dictate the nature of the composition ultimately dispensed by the system 200. For example, the consistency of the composition may be controlled by the pH induced thickener included in the composition and/or the pH of the composition. According to the user selections, the selection module 206 may provide an accurate and efficient means of dispensing the respective compositions 102, 104, 108 and parts which constitute the composition.

In particular, the parameters may include selection of a flavoring agent or flavoring agents. As previously noted, the possibility of including the flavoring agent in, for instance, the second separate part may enable more flexibility in terms of the flavor options because providing the flavoring agent separately in this manner reduces the risk that the flavoring agent is degraded by other ingredient(s) of the kit 100, such as the peroxide compound over a relatively long storage period/shelf life. The system 200 may therefore permit the user to select the flavor(s) of the composition dispensed by the system 200 according to personal preference, in a manner similar to soft drink flavor selection using a soda fountain.

In a simple example, the user may select either to include the flavoring agent in the composition, e.g. by opting to include the second separate part in the composition, or not to include the flavoring agent in the composition. In a more elaborate example, the user may select from different flavoring agents included in the kit 100. The user interface 204 may, for instance, take the form of a touchscreen to enable user-friendly parameter selection. Since flavor and/or smell may play an important role in consumer product preference, touchscreen options of peroxide based gel vending machine may be designed as flavor or fragrance choices.

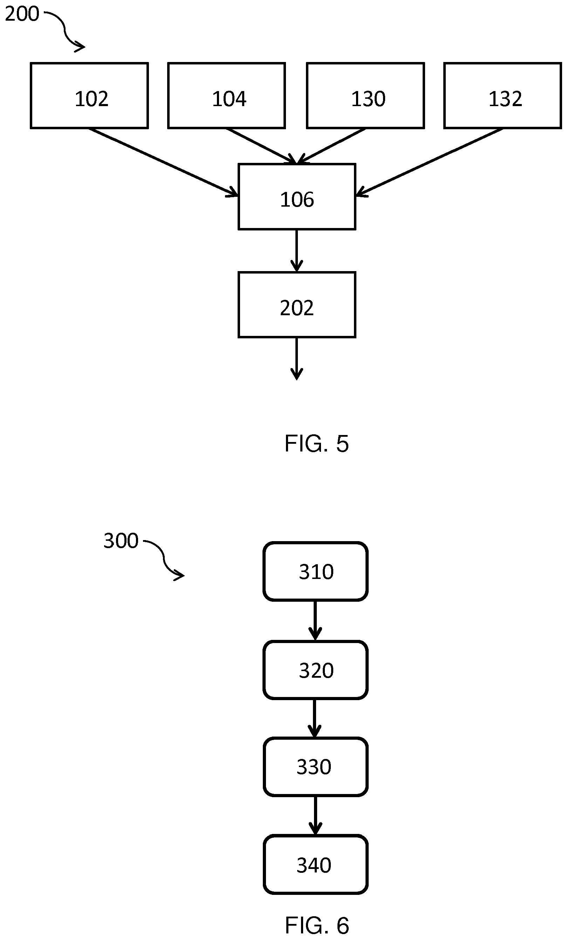

FIG. 5 shows a block diagram of a system 200 according to an embodiment. In this non-limiting example, the system 200 includes the first aqueous composition 102 and the second composition 104. Moreover, the first separate part 130 and the second separate part 132 are included in the system 200. The dispensing pump 202 dispenses the composition from the mixing chamber 106.

Any suitable mixing ratio for the first aqueous composition 102, the second composition 104 and the third composition 108 may be employed, according to the desired peroxide content, pH and the content of any other functional ingredients which are to be included in the composition as long as the required peroxide composition pH is within the required range using a particular set of kit compositions. The mixing ratios can be chosen as disclosed herein before.

The mixing ratio of each of the compositions of the kit or system may be determined by the desired characteristics of the peroxide composition, in relation to its intended purpose, e.g. tooth whitening or sanitization. This system 200 may accurately control dosage of each of the respective compositions 102, 104, 108 and parts 130, 132. Each of the compositions 102, 104, 108 and parts 130, 132 may be, for instance, supplied in cartridge form to facilitate dosing control and replacement of the compositions 102, 104, 108 and parts 130, 132 in the system 200. Such a replaceable cartridge-based system 200 may further facilitate the flavoring agent selection described above.

The kit examples and the described devices and systems are preferably designed to accommodate any of the previously described kits with their compositions and other ingredients in the specified amounts.

FIG. 6 shows a flowchart of a method 300 according to an embodiment. The method 300 is for preparing a peroxide composition having a pH in the range of 7 to 12. In step 310, a first aqueous composition comprising a peroxide agent is provided e.g bought from a vendor or custom made. The first aqueous composition has a first pH of less than 7 for reasons as previously described.

In step 320, a second composition comprising a basic agent is provided e.g. from a vendor or custom made.

A pH induced thickener as described herein before is provided in step 330. It may be obtained from a vendor or be custom made. The pH induced thickener maybe dissolved and/or dispersed in a solvent or dispersion medium at the first pH and/or be in dry form. Many of the polymers may be obtained as acidic emulsions from different vendors and these may be used as is when the pH has a value in line with the first pH.

In step 340, the first aqueous composition and the second composition, together with the pH induced thickener, are combined preferably mixed to form the peroxide composition having the alkaline pH in the range of 7 to 12. Accordingly the peroxide composition thickens (e.g. because it forms a gel) to provide the advantages described herein before.

The combining 340 may comprise forming a pre-combination of the first aqueous composition and the pH induced thickener. The pre-combination may then be combined with the second composition, thereby to afford the peroxide composition. Initial forming of the pre-combination of the first aqueous composition and the pH induced thickener may permit effective, e.g. intimate, mixing of the ingredients contained in the first component, such as e.g. the peroxide agent, with the pH induced thickener so that subsequent mixing of the pre-combination with the second composition may afford a gel-like composition in situ in which the ingredients of the first aqueous composition are uniformly mixed.

The peroxide composition and thus the kit and system therefore may be formulated to be suitable for tooth whitening, and may thus be suitable for retail, take-home or chairside-type tooth whitening products in the oral healthcare and cosmetics sectors. The peroxide composition and thus the kit and system therefore may also be formulated for use in sanitization applications, e.g. when relatively long sanitizing times during which the gel-like composition is applied to surfaces are required. The peroxide composition and kit and system therefore may be employed for sanitizing surfaces (including human body surfaces, such as the hand, foot, etc., when applicable) at, for instance, the dental office, hospital, nursing home, sports venue, food-processing plant, etc. The kit 100 and system may be designed, for instance, into a vending machine-like system 200, as described above, which may be placed at those facilities.

In summary, provided is a kit or system (100) for preparing a peroxide composition with increased viscosity with a pH in the range of 7 to 12 in an almost instantaneous method. For example, the kit or system may be used to provide an almost instantly obtainable (hydro)gelled peroxide composition. The system comprises a first aqueous composition (102) comprising a peroxide agent. The first aqueous composition has a first pH of less than 7. The system further comprises a second composition (104) comprising a basic agent. The second composition is separate from the first aqueous composition. A pH induced thickener is included in the kit or system separate from the second composition. The peroxide composition is obtainable by combining the parts of the kit or system. The pH induced thickener is capable of increasing the viscosity of the peroxide composition upon subjecting it to the pH in the range of 7 to 12. The kit or system has sufficient basic agent for the peroxide composition to cause the pH in the range of 7 to 12. A method for preparation and use of the kit or system is also provided.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A system (100) for preparing a peroxide composition, the system comprising:
- a first quantity of a first composition (102) comprising a peroxide agent and water and having a first pH lower than 7;
- a second quantity of a second composition (104) separate from said first composition, the second quantity comprising an amount of a basic agent;
- an amount of a pH induced thickener capable of thickening an aqueous composition upon an increase of the pH of this aqueous composition from an acidic to an alkaline value, the pH induced thickener being present in the system in at least one of:
- a solvent and/or dispersion medium free form; and
- a solvent and/or dispersion medium containing form in which the pH induced thickener is dissolved and/or dispersed at the first pH,
- the peroxide composition being obtainable by combining the first quantity of the first composition, the second quantity of the second composition and the amount of pH induced thickener; and
- the amount of the basic agent being sufficient for causing the peroxide composition to have a composition pH in the range of 7 to 12.

2. The system according to claim 1, wherein the pH induced thickener comprises or consists of one or more polymers each comprising monomer residues wherein at least a fraction of the monomer residues comprises acidic groups.

3. The system of claim 2 wherein the acidic groups comprise or consist of one or more groups chosen from carboxylic acid groups, sulfonic acid groups, and phosphoric acid groups.

4. The system according to any one of the claims 1 to 3, wherein the pH induced thickener comprises or consists of one or more alkali soluble emulsion (ASE) polymers and/or one or more hydrophobically-modified alkali-soluble emulsion (HASE) polymers.

5. The system according to any of the claims 2 to 4 wherein the pH induced thickener comprises one or more acrylic co-polymers having the acidic groups at least in the form of carboxylic acid groups.

6. The system (100) according to any one of the claims 1 to 5, wherein the pH induced thickener comprises one or more cellulose polymers having residues at least a fraction of which comprise hydroxyl groups.

7. The system (100) according to any one of the previous claims, wherein the first composition (102) comprises the pH induced thickener in the solvent and/or dispersion medium containing form.

8. The system according to any one of claims 1 to 6, wherein the system comprises a third quantity of a third composition separate from the first composition and the third composition comprises at least a part and preferably all of the pH induced thickener.

9. The system according to any of the previous claims wherein the second composition comprises water and has a second pH higher than 7.

10. The system according to any one of the previous claims, wherein the first quantity and the second quantity are sufficient to mix them in a ratio of the first quantity to the second quantity in a range of 10 to 0.1, preferably in the range of 5 to 0.2, or most preferably in the range of 5 to 0.8.

11. The system according to any one of the previous claims, wherein the first composition comprises a solvent and/or dispersion medium and has a viscosity lower than 1000 mPa·s, preferably lower than 500 mPa·s, most preferably lower than 100 mPa·s at standard conditions.

12. The system according to any of claims 1 to 11, wherein the amount of the pH induced thickener is sufficient for causing the peroxide composition to have a viscosity higher than 50000 mPa·s, more preferably higher than 100000 mPa·s at standard conditions.

13. The system (100) according to any of the previous claims, wherein the first pH is in the range of 2 to 5 and/or, when the system comprises the third composition having a third pH, the third pH is in the range of 2 to 5.

14. The system (100) according to any of the previous claims, comprising one or more of:
- a tooth desensitizing agent;
- a tooth remineralizing agent;
- a flavouring agent;

15. The system (100) according to any of the previous claim, comprising a multicompartment syringe (114), said syringe including at least:
- a first compartment (116) containing the pH induced thickener;
- a second compartment (118), containing the second composition (104);
- a mixing compartment (120) attachable to or attached to the syringe for receiving and mixing the contents of at least the first compartment and the second compartment and having an outlet (122) for permitting the peroxide composition to exit the syringe upon use of the syringe.

16. The system according to any of the claims 1 to 14, comprising a dispensing system (200) for dispensing the peroxide composition, the dispensing system comprising:
- a mixing unit for combining quantities of the first composition, the second composition and the PH induced thickener to therewith provide the peroxide composition;
- a user interface (204) for enabling a user to provide input parameters relating to at least one of:
- use of the peroxide composition;
- a peroxide agent content in the peroxide composition;
- the composition pH;
- the viscosity of the peroxide composition; and
- flavor and/or smell of the peroxide composition;
- a selection module (206) for controlling the mixing unit to determine the quantities of components to be combined for the peroxide composition based on the input parameters.

17. The system according to any one of claims 1 to 16, for providing the peroxide composition for use as an orally applicable tooth whitening composition or a disinfectant composition in the prevention or treatment of oral infections.

18. Use of a system according to any one of the claims 1 to 17, for preparing a peroxide composition having a composition pH in the range of 7 to 12, preferably in the range of 7 to 9.5.

19. Use according to claim 18, wherein the peroxide composition is an orally applicable tooth whitening composition or a disinfectant composition for use in the prevention or treatment of oral infections.

20. A peroxide composition having a composition pH in the range of 7 to 12, preferably in the range of 7 to 9.5, comprising a peroxide agent, water, a basic agent and an amount of a pH induced thickener capable of thickening an aqueous composition upon an increase of the pH of this aqueous composition from an acidic to an alkaline value.

21. A peroxide composition as claimed in claim 20 obtainable or obtained by combining or mixing a first quantity of the first composition, a second quantity of the second composition and the pH induced thickener as defined in any of the system claims 1 to 17.

22. A method (300) for preparing a peroxide composition having a pH in the range of 7 to 12, the method comprising the step of combining:
- a first quantity of a first composition (102) comprising a peroxide agent and water and having a first pH lower than 7;
- a second quantity of a second composition (104) separate from said first composition, the second quantity comprising an amount of a basic agent;
- an amount of pH induced thickener capable of thickening a composition including water upon an increase of the pH of this composition from an acidic to an alkaline value, the pH induced thickener before combining having at least one of:
- a solvent and/or dispersion medium free form; and
- a solvent and/or dispersion medium containing form in which the pH induced thickener is dissolved and/or dispersed at a first pH lower than 7.
